# EUROPÄISCHE PATENTSCHRIFT

(11) **EP 2 396 337 B1**
(45) Veröffentlichungstag und Bekanntmachung des Hinweises auf die Patenterteilung: **13.07.2016**
(21) Anmeldenummer: 10703859.8
(22) Anmeldetag: 11.02.2010
(51) Int. Cl.: C07F 3/00, C07D 211/10

(54) **HERSTELLUNG UND VERWENDUNG VON ZINKAMIDEN**
PREPARATION AND USE OF ZINC-AMIDES
PERPARATION ET UTLISATION DES AMIDES DU ZINC

(30) Priorität: 13.02.2009 EP 09100112
(43) Veröffentlichungstag der Anmeldung: 21.12.2011
(73) Patentinhaber: Ludwig-Maximilians-Universität München, 80539 München (DE)
(72) Erfinder: KNOCHEL, Paul, 81475 München (DE); MOSRIN, Marc, 80337 München (DE)
(74) Vertreter: Rottmayer, Hans
(86) Internationale Anmeldenummer: PCT/EP2010/051677
(87) Internationale Veröffentlichungsnummer: WO 2010/092096

(56) Entgegenhaltungen:
- JP-A- 2004 010 585
- KONDO Y ET AL: "TMP-Zincate as Highly Chemoselective Base for Directed Ortho Metalation", JOURNAL OF THE AMERICAN CHEMICAL SOCIETY, AMERICAN CHEMICAL SOCIETY, US, vol. 121, 25 March 1999 (1999-03-25), pages 3539-3540, XP002490915, ISSN: 0002-7863, DOI: 10.1021/JA984263T [retrieved on 1999-03-25]
- TATSUSHI IMAHORI ET AL: 'REGIOCONTROLLED DEPROTONATIVE-ZINCATION OF BROMOPYRIDINES USING AMINOZINCATES' CHEMICAL COMMUNICATIONS - CHEMCOM Nr. 23, 22 November 2001, GB, Seiten 2450 - 2451, XP055238147 DOI: 10.1039/b108252n ISSN: 1359-7345

## Beschreibung

Die direkte Metallierung aromatischer und heterozyklischer Verbindungen ist ein wichtiges Verfahren zur Funktionalisierung von Grundgerüsten. Für die *ortho*-Metallierung von ungesättigten Systemen werden in großem Umfang Lithiumbasen verwendet ((a) Snieckus, V. Chem. Rev. 1990, 90, 879. (b) Clayden, J.; Stimson, C. C.; Keenan M. Chem. Comm. 2006, 1393. (c) Schlosser M. Angew. Chem. Int. Ed. 2005, 44, 376. (d) Henderson, K. W.; Kerr, W. J. Chem. Eur. J. 2001, 3431. (e) Turck, A.; Ple, N.; Mongin, F.; Queguiner, G. Tetrahedron 2001, 57, 4489. (f) Mongin F.; Queguiner, G. Tetrahedron 2001, 57, 4059. (g) Levoux, F.; Jeschke, P.; Schlosser, M. Chem. Rev. 2005, 105, 827. (h) Kauch, M.; Hoppe, D. Synthesis 2006, 1578. (i) Clegg, W.; Dale, S. H.; Hevia, E.; Honeyman, G. W.; Mulvey R. E. Angew. Chem. Int. Ed. 2006, 45, 2371. (j) Hodgson, D. M.; Miles, S. M. Angew. Chem. Int. Ed. 2006, 45, 93. (k) Yus, M.; Foubelo, F. Handbook of Functionalized Organometallics, Knochel, P. Hrsg., Wiley-VCH: Weinheim, Germany 2005; Bd. 1, Seite 7). Die Verwendung von Magnesiumbasen ((a) Eaton, P. E.; Martin, R. M. J. Org. Chem. 1988, 53, 2728. (b) Eaton, P. E.; Lee, C.-H.; Xiong, Y. J. Am. Chem. Soc. 1989, 111, 8016. (c) Eaton, P. E.; Lukin, K. A. J. Am. Chem. Soc. 1993, 115, 11370. (d) Zhang, M.-X.; Eaton, P. E. Angew. Chem. Int. Ed. 2002, 41, 2169) findet erneut großes Interesse ((a) Hevia, E.; Honeyman, G. W.; Kennedy, A. R.; Mulvey, R. E.; Sherrington, D. C. Angew. Chem. Int. Ed. 2005, 44, 68. (b) Andrikopolous, P. C.; Armstrong, D. R.; Graham, D. V.; Hevia, E.; Kennedy, A. R.; Mulvey, R. E.; O'Hara, C. T.; Talmard, C. Angew. Chem. Int. Ed. 2005, 44, 3459. (c) Kondo, Y.; Akihiro, Y.; Sakamoto, T. J. Chem. Soc., Perkin Trans. 1 1996, 2331. (d) Shilai, M.; Kondo, Y.; Sakamoto, T. J. Chem. Soc., Perkin Trans. 1 2001, 442. (e) Bayh, O.; Awad, H.; Mongin, F.; Hoarau, C.; Bischoff, L.; Trécourt, F.; Queguiner, G.; Marsais, F.; Blanco, F.; Abarca, B.; Ballesteros, R. J. Org. Chem. 2005, 70, 5190. (f) Eaton, P. E.; Zhang, M.-X.; Komiya, N.; Yang, C.-G.; Steele, I.; Gilardi, R. Synlett 2003, 9, 1275). Auch für Lithium Magnesiate gibt es nützliche Syntheseanwendungen ((a) Kitagawa, K.; Inoue, A.; Shinokubo, H.; Oshima, K. Angew. Chem. Int. Ed. 2000, 39, 2481. (b) Farkas, J.; Stoudt, S. J.; Hannawalt, E. M.; Pajeski, A. D.; Richey, H. G. Organometallics 2004, 23, 423. (c) Awad, H.; Mongin, F.; Trecourt, F.; Queguiner, G.; Marsais, F.; Blanco, F.; Abarca, B.; Ballesteros, R. Tetrahedron Lett. 2004, 45, 6697; (a) Garcia-Alvarez, P.; Graham, D. V.; Hevia, E.; Kennedy, A. R.; Klett, J.; Mulvey, R. E.; O'Hara, C. T.; Weatherstone, S. Angew. Chem. Int. Ed. 2008, 47, 8079. (b) Mulvey, R. E. Organometallics 2006, 25, 1060. (c) Mulvey, R. E. Chem. Comm. 2001, 1049. (d) Westerhausen, M. Dalton Trans. 2006, 4755. (e) Mulvey, R. E.; Mongin, F.; Uchiyama, M.; Kondo, Y. Angew. Chem. Int. Ed., 2007, 46, 3802). Gemischte Mg/Li-Basen des Typs R₂NMgCl·LiCl wie 2,2,6,6-Tetramethylpiperidid-Magnesiumchlorid-Lithiumchlorid (TMPMgCl·LiCl; Turbo-Hauser-Base) erweisen sich als besonders wirksame Metallierungsmittel, die mit funktionellen Gruppen wie einem Ester, Nitril oder Arylketon kompatibel sind ((a) Krasovskiy, A.; Krasovskaya, V.; Knochel, P. Angew. Chem. Int Ed. 2006, 45, 2958. (b) Lin, W.; Baron, O.; Knochel, P. Org. Lett. 2006, 8, 5673. (c) Mosrin, M.; Knochel, P. Org. Lett 2008, 10, 2497. (d) Mosrin, M.; Boudet, N.; Knochel, P. Org. Biomol. Chem. 2008, 6, 3237. (e) Clososki, G. C.; Rohbogner, C. J.; Knochel, P. Angew. Chem. Int. Ed. 2007, 46, 7681. (f) Rohbogner, C. J.; Clososki, G. C.; Knochel, P. Angew. Chem. Int. Ed. 2008, 47, 1503). Empfindlichere funktionelle Gruppe wie eine Aldehyd- oder eine Nitrogruppe werden aber nicht toleriert. Auch bei empfindlichen Heterozyklen kann es zu Fragmentierung kommen ((a) Micetich, R. G. Can. J. Chem. 1970, 48, 2006. (b) Meyers, A. I; Knaus, G. N. J. Am. Chem. Soc. 1974, 95, 3408. (c) Knaus, G. N.; Meyers, A. I. J. Org. Chem. 1974, 39, 1189. (d) Miller, R. A.; Smith, M. R.; Marcune, B. J. Org. Chem. 2005, 70, 9074. (e) Hilf, C.; Bosold, F.; Harms, K.; Marsch, M.; Boche, G. Chem. Ber. Rec. 1997, 130, 1213). Deshalb wird von einer Reihe Zinkamiden berichtet, die nach Metallierung zinkorganische Reagenzien liefern, die mit den meisten funktionellen Gruppen kompatibel sind. In einer wegweisenden Arbeit berichtet Kondo, dass Lithium-di-tert.-butyl-(2,2,6,6-tetra-methylpiperidino)zinkat (Li*t*-Bu₂TMPZn) eine ausgezeichnete Base für die Zinkierung verschiedener Aromaten ist ((a) Micetich, R. G. Can. J. Chem. 1970, 48, 2006. (b) Meyers, A. I.; Knaus, G. N. J. Am. Chem. Soc. 1974, 95, 3408. (c) Knaus, G. N.; Meyers, A. I. J. Org. Chem. 1974, 39, 1189. (d) Miller, R. A.; Smith, M. R.; Marcune, B. J. Org. Chem. 2005, 70, 9074. (e) Hilf, C.; Bosold, F.; Harms, K.; Marsch, M.; Boche, G. Chem. Ber. Rec. 1997, 130, 1213). Leider ist die Verwendung hochreaktiver Zinkate und verwandter at-Basen ((a) Uchiyama, M.; Matsumoto, Y.; Nobuto, D.; Furuyama, T.; Yamaguchi, K.; Morokuma, K. J. Am. Chem. Soc. 2006, 128, 8748. (b) Clegg, W.; Dale, S. H.; Drummond, A. M.; Hevia, E.; Honeyman, G. W.; Mulvey, R. E. J. Am. Chem. Soc. 2006, 128, 7434. (c) Hevia, E.; Honeyman, G. W.; Mulvey, R. E. J. Am. Chem. Soc. 2005, 127, 13106. (d) Armstrong, D. R.; Clegg, W.; Dale, S. H.; Hevia, E.; Hogg, L. M.; Honeyman, G. W.; Mulvey, R. E. Angew. Chem. Int. Ed. 2006, 45, 3775. (e) Clegg, W.; Dale, S. H.; Harrington, R. W.; Hevia, E.; Honeyman, G. W.; Mulvey, R. E. Angew. Chem. Int. Ed. 2006, 45, 2374. (f) Naka, H.; Uchiyama, M.; Matsumoto, Y.; Wheatly, A. E. H.; McPartlin, M.; Morey, J. V.; Kondo, Y. J. Am. Chem. Soc. 2007, 129, 1921) nicht kompatibel mit empfindlichen Funktionen wie einem Aldehyd oder einer Nitrogruppe. Wir haben kürzlich die Herstellung einer hoch chemoselektiven Base TMP₂Zn·2MgCl₂·2LiCl für die direkte Zinkierung empfindlicher Aromaten und Heteroaromaten beschrieben ((a) Wunderlich, S. H.; Knochel, P. Angew. Chem. Int. Ed. 2007, 46, 7685. (b) Mosrin, M.; Knochel P. Chem. Eur. J. 2009**,** DOI: 10.1002/chem.200801831). Einige elektronenarme funktionalisierte Arene und Heteroarene geben mit diesem Reagenz bezüglich Ausbeute und der Reaktionsselektivität keine zufrieden stellenden Ergebnisse. Auch müssen verschiedene aktivierte Aromaten oder Heteroaromaten, wie Nitroderivate oder Pyridazine bei unter -50 °C metalliert werden, was für größere Reaktionsmaßstäbe schlecht geeignet ist ((a) Wunderlich, S. H.; Knochel, P. Angew. Chem. Int. Ed. 2007, 46, 7685. (b) Mosrin, M.; Knochel P. Chem. Eur. J. 2009**,** DOI: 10.1002/chem.200801831; Wunderlich, S. H.; Knochel, P. Chem. Comm. 2008, 47, 6387).

Ein Reagenz der allgemeinen Formel

R¹R²N-ZnY LiY (I)

worin ist:
R¹, R² unabhängig ausgewählt aus H, substituiertem oder unsubstituiertem Aryl oder Heteroaryl mit ein oder mehr Heteroatomen, geradem, verzweigtem oder zyklischem substituiertem oder unsubstituiertem Alkyl, Alkenyl, Alkinyl oder deren Silylderivaten; wobei R¹ und R² zusammen Teil einer zyklischen oder polymeren Struktur sein können, worin mindestens einer der Reste R¹ und R² nicht H ist;
Y ausgewählt aus der Gruppe mit F; Cl; Br; I; CN; SCN; NCO; HalOₙ; wobei n gleich 3 oder 4 und Hal ausgewählt ist aus Cl, Br und I; NO₃; BF₄; PF₆; H; einem Carboxylat der allgemeinen Formel R^{x}CO₂; einem Alkoholat der allgemeinen Formel OR^{x}; einem Thiolat der allgemeinen Formel SR^{x}; R^{x}P(O)O₂; oder SCOR^{x}; oder SCSR^{x}; OₙSR^{x}; wobei n gleich 2 oder 3; oder NOₙ, wobei n gleich 2 oder 3; und einem Derivat davon; worin R^{x} ein substituiertes oder unsubstituiertes Aryl oder Heteroaryl mit ein oder mehr Heteroatomen, gerades, verzweigtes oder zyklisches substituiertes oder unsubstituiertes Alkyl, Alkenyl, Alkinyl oder deren Derivate oder H ist;
oder als Addukt mit einem Lösungsmittel.

R¹, R² sind zyklisch und mit R³ und R⁴ substituiert, die unabhängig ausgewählt sind aus H, substituiertem oder unsubstituiertem Aryl oder Heteroaryl mit ein oder mehr Heteroatomen, geradem, verzweigtem oder zyklischem substituiertem oder unsubstituiertem Alkyl, Alkenyl, Alkinyl oder deren Silylderivaten; und R¹ und R² zusammen oder R³ und R⁴ zusammen können einen Teil einer zyklischen oder polymeren Struktur bilden; wobei mindestens einer der Reste R¹ und R² und mindestens einer der Rest R³ und R⁴ nicht H ist.

Das Reagenz R¹R²N-ZnY LiY (I) ist vorzugsweise 2,2,6,6-Tetramethylpiperid-Zinkchlorid-Lithiumchlorid oder die Lösung des Reagenz in einem Lösungsmittel. Das Lösungsmittel ist polar und aprotisch. Vorzugsweise ist das Lösungsmittel ausgewählt aus zyklischen, geraden oder verzweigten Mono- oder Polyethern, Thioethern, Aminen, Phosphinen und deren Derivaten, die ein oder mehrere weitere Heteroatome enthalten, ausgewählt aus O, N, S und P, vorzugsweise Tetrahydrofuran (THF), 2-Methyltetrahydrofuran, Dibutylether, Diethylether, tert.-Butylmethylether, Dimethoxyethan, Dioxanen, vorzugsweise 1,4-Dioxan, Triethylamin, Ethyldiisopropylamin, Dimethylsulfid, Dibutylsulfid; zyklischen Amiden, vorzugsweise N-Methyl-2-pyrrolidon (NMP), N-Ethyl-2-pyrrolidon (NEP), N-Butyl-2-pyrrolidon (NBP); zyklischen, geraden oder verzweigten Alkanen und/oder Alkenen, wobei ein oder mehr Wasserstoffatome durch ein Halogenatom ersetzt sind, vorzugsweise Dichlormethan, 1,2-Dichlorethan, Tetrachlormethan (CCl₄); Harnstoffderivaten, vorzugsweise N,N'-Dimethylpropylenharnstoff (DMPU); aromatischen, heteroaromatischen oder aliphatischen Kohlenwasserstoffen, vorzugsweise Benzol, Toluol, Xylol, Pyridin, Pentan, Cyclohexan, Hexan, Heptan; Hexamethylphosphortriamid (HMPA), Kohlenstoffdisulfid (CS₂); oder deren Kombinationen.

Das erfindungsgemäße Verfahren zur Herstellung eines gemischten Zn/Li-Amids umfasst die Umsetzung eines primären oder sekundären Amins mit einem Lithiumalkyl in einem Lösungsmittel.

Es umfasst vorzugsweise das Verfahren zur Herstellung eines Reagenzes der allgemeinen Formel

R¹R²N-ZnY LiY (I)

worin ist
R¹, R² unabhängig ausgewählt aus H, substituiertem oder unsubstituiertem Aryl oder Heteroaryl mit ein oder mehr Heteroatomen, geradem, verzweigtem oder zyklischem substituiertem oder unsubstituiertem Alkyl, Alkenyl, Alkinyl oder deren Siliciumderivaten; und R¹ und R² zusammen ein Teil einer zyklischen oder polymeren Struktur sein können; und worin mindestens einer der Reste R¹ und R² nicht H ist;
Y ausgewählt aus der Gruppe mit F; Cl; Br; I; CN; SCN; NCO; HalOₙ, wobei n gleich 3 oder 4 und Hal ausgewählt ist aus Cl, Br und I; NO₃; BF₄; PF₆; H; einem Carboxylat der allgemeinen Formel R^{x}CO₂; einem Alkoholat der allgemeinen Formel OR^{x}; einem Thiolat der allgemeinen Formel SR^{x}; R^{x}P(O)O₂; oder SCOR^{x}; oder SCSR^{x}; OₙSR^{x}, wobei n gleich 2 oder 3; oder NOₙ, wobei n gleich 2 oder 3; und einem Derivat davon; wobei R^{x} ein substituiertes oder unsubstituiertes Aryl oder Heteroaryl mit ein oder mehr Heteroatomen, gerades, verzweigtes oder zyklisches substituiertes oder unsubstituiertes Alkyl, Alkenyl, Alkinyl oder deren Derivate oder H ist; und die Umsetzung in einem Lösungsmittel von R¹R²N-H mit R^{x}Li in Gegenwart von ZnY₂ erfolgt und X wie oben Y definiert ist.

X und Y sind unabhängig oder beide Cl, Br oder I und vorzugsweise Cl. Das Lithium-organyl-Reagenz ist sek.-Butyl-Li und das Lösungsmittel ist ausgewählt aus zyklischen, geraden oder verzweigten Mono- oder Polyethern, Thioethern, Aminen, Phosphinen und deren Derivaten, die ein oder mehr weitere Heteroatome enthalten, ausgewählt aus O, N, S und P, vorzugsweise Tetrahydrofuran (THF), 2-Methyltetrahydrofuran, Dibutylether, Diethylether, tert.-Butylmethylether, Dimethoxyethan, Dioxanen, vorzugsweise 1,4-Dioxan, Triethylamin, Ethyldiisopropylamin, Dimethylsulfid, Dibutylsulfid; zyklischen Amiden, vorzugsweise N-Methyl-2-pyrrolidon (NMP), N-Ethyl-2-pyrrolidon (NEP), N-Butyl-2-pyrrolidon (NBP); zyklischen, geraden oder verzweigten Alkanen und/oder Alkenen, wobei ein oder mehr Wasserstoffatome durch ein Halogenatom ersetzt sind, vorzugsweise Dichlormethan, 1,2-Dichlorethan, CCl₄; Harnstoffderivaten, vorzugsweise N,N'-Dimethylpropylenharnstoff (DMPU); a-romatischen, heteroaromatischen oder aliphatischen Kohlenwasserstoffen, vorzugsweise Benzol, Toluol, Xylol, Pyridin, Pentan, Cyclohexan, Hexan, Heptan; Hexamethylphosphortriamid (HMPA), CS₂; oder deren Kombinationen.

Wenn nicht anders definiert, bedeuten alle hier verwendeten technischen und wissenschaftlichen Begriffe das, was der Durchschnittsfachmann auf dem Gebiet dieser Erfindung darunter versteht. Alle hier erwähnten Veröffentlichungen und anderen Bezugsstellen sind hier durch Bezugnahme vollinhaltlich aufgenommen.

Wie hier verwendet, bezeichnen die Begriffe "Alkyl", "Alkenyl" und "Alkinyl" gerade, zyklische und verzweigte substituierte und unsubstituierte C₁-C₂₀-Verbindungen. Bevorzugte Bereiche für diese Verbindungen sind C₁₋C₁₀, vorzugsweise C₁-C₅ (Niederalkyl) und C₂-C₁₀ bzw. vorzugsweise C₂-C₅ für Alkenyl und Alkinyl. Der Begriff "Cycloalkyl" bezeichnet im Allgemeinen gerade und verzweigte substituierte und unsubstituierte C₃-C₂₀-Cycloalkane. Hier sind bevorzugte Bereiche C₃-C₁₅, stärker bevorzugt C₃-C₈.

Wenn einer der Reste R¹, R², R³ und/oder R⁴ mit einem Substituenten substituiert ist, kann der Substituent von einem Fachmann aus jedem bekannten Substituenten ausgewählt werden. Ein Fachmann wählt einen möglichen Substituenten nach seinem Wissen und ist in der Lage, einen Substituenten auszuwählen, der andere im Molekül vorhandene Substituenten oder mögliche Reaktionen nicht stört, insbesondere nicht die in dieser Anmeldung beschriebenen Reaktionen. Mögliche Substituenten sind u. a. ohne Beschränkung:
- Halogene, vorzugsweise Fluor, Chlor, Brom und lod;
- aliphatische, alizyklische, aromatische oder heteroaromatische Kohlenwasserstoffe, insbesondere Alkane, Alkylene, Arylene, Alkylidene, Arylidene, Heteroarylene und Heteroarylidene; Carbonsäuren einschließlich deren Salzen;
- Carbonsäurehalogenide;
- aliphatische, alizyklische, aromatische oder heteroaromatische Carbonsäureester;
- Aldehyde;
- aliphatische, alizyklische, aromatische oder heteroaromatische Ketone;
- Alkohole und Alkoholate, einschließlich einer Hydroxylgruppe;
- Phenole und Phenolate;
- aliphatische, alizyklische, aromatische oder heteroaromatische Ether;
- aliphatische, alizyklische, aromatische oder heteroaromatische Peroxide;
- Hydroperoxide;
- aliphatische, alizyklische, aromatische oder heteroaromatische Amide oder Amidine;
- Nitrile;
- aliphatische, alizyklische, aromatische oder heteroaromatische Amine;
- aliphatische, alizyklische, aromatische oder heteroaromatische Imine;
- aliphatische, alizyklische, aromatische oder heteroaromatische Sulfide, einschließlich einer Thiolgruppe;
- Sulfonsäuren einschließlich deren Salze;
- Thiole und Thiolate;
- Phosphonsäuren einschließlich deren Salze;
- Phosphinsäuren einschließlich deren Salze;
- Phosphonsäuren, einschließlich deren Salze; Phosphinsäuren, einschließlich deren Salze.

Die Substituenten können an die Reste R¹, R², R³ und/oder R⁴ über ein Kohlenstoffatom, ein Sauerstoffatom, ein Stickstoffatom, ein Schwefelatom oder ein Phosphoratom gebunden werden. Die Heteroatome in jeder Gruppe, die Heteroatome enthält, wie z. B. Heteroarylene oder Heteroaromaten, können vorzugsweise N, O, S und P sein.

Wenn R¹ und R² oder R³ und R⁴ ein Teil einer zyklischen Struktur sind, ist selbstverständlich, dass R¹ und R² zusammen oder R³ und R⁴ zusammen ein zweiwertiges gesättigtes oder ungesättigtes gerades oder verzweigtes Alkyl, Alkenyl oder Alkinyl sind, das in Verbindung mit dem Stickstoffatom des Amids ein zyklisches sekundäres Amid bildet. Ein Beispiel für ein solches zyklisches Amid ist das Amid von TMPH. Außerdem können die Reste R¹ und R² und/oder R³ und R⁴ Teil einer polymeren Struktur sein. Das Stickstoffatom des Amids ist mit einem Polymer-Grundgerüst verbunden, das sogar mehr als ein Stickstoffatom für die Bildung eines erfindungsgemäßen Amids enthalten kann.

Der Begriff "Aryl", wie hier verwendet, bezeichnet ein substituiertes oder unsubstituiertes C₄-C₂₄-Aryl. Mit "Heteroaryl" ist ein substituiertes oder unsubstituiertes C₃-C₂₄-Aryl gemeint, das ein oder mehr Heteroatome enthält, wie B, O, N, S, Se, P. Bevorzugte Bereiche für beide sind C₄-C₁₅, stärker bevorzugt C₄-C₁₀, und umfasst sind Aryle und kondensierte Aryle mit oder ohne Heteroatome. Eine bevorzugte Ringgröße enthält 5 oder 6 Ringatome.

Wir haben zum Beispiel die Herstellung einer selektiveren Zinkbase untersucht, wodurch chemoselektive Metallierungen bei 25 °C für die direkte Zinkierung empfindlicher Aryl- und Heteroarylsubstrate möglich sind. Die Behandlung von 2,2,6,6-Tetramethylpiperidin (1; TMP-H) mit n-BuLi (1,0 Äquiv., -40 bis -10 °C, 1 Std.) und die anschließende Zugabe von ZnCl₂ (1,1 Äquiv., -10 °C, 30 Minuten) liefert eine etwa 1,3 M Lösung von TMPZnCl·LiCl (2), die bei Raumtemperatur stabil ist (Schema 1). Im Gegensatz zu TMP₂Zn·2MgCl₂·2LiCl zeigt diese komplexe Base eine sehr gute Chemoselektivität für die Zinkierung bei 25 °C von verschiedenen sensitiven Aromaten und Heterozyklen.

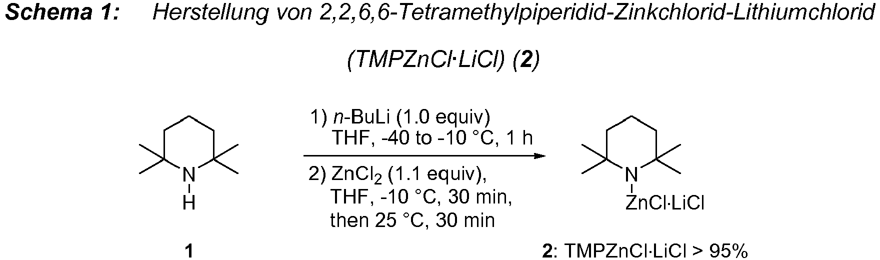

Das erfindungsgemäße Reagenz konnte in einer Reaktion mit einem Elektrophil vorzugsweise für die Deprotonierung eines beliebigen Substrats, das stabilisierte oder unstabilisierte Carbanionen bilden kann, verwendet werden.

Beispiele für die Verwendung des erfindungsgemäßen Reagenzes geben die folgenden Tabellen.

**Tabelle 1: Produkte, die durch regio- und chemoselektive Zinkierung von Diazinen des Typs 3, 6 und 9 mit TMPZnCl·LiCl (2; 1,1 Äquiv.; 25 °C) und Quenching mit Elektrophilen erhalten wurden**

| entry | substrate of type **3, 6** and **9** | electrophile | product | yield, %^{a} |
|---|---|---|---|---|
| 1 | 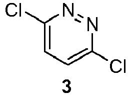 | I₂ | 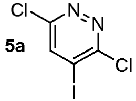 | 84 |
| 2 | **3** | 4-fluorobenzoyl chloride | 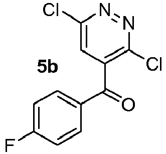 | 96^{b} |
| 3 | **3** | 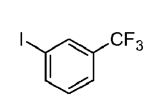 | 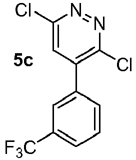 | 83^{c} |
| 4 | 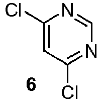 | I₂ | 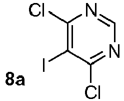 | 83 |
| 5 | **6** | furoyl chloride | 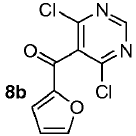 | 71^{b} |
| 6 | **6** | allyl bromide | 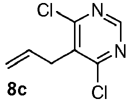 | 89^{d} |
| 7 | 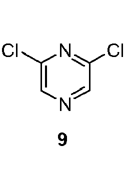 | I₂ | 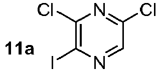 | 90 |
| 8 | **9** | 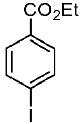 | 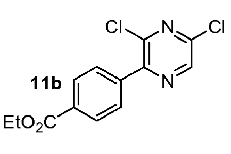 | 87^{c} |
| 9 | **9** | 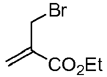 | 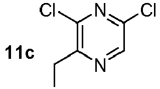 | 72^{d} |

| | | | | |
|---|---|---|---|---|
| ^{a} Isoliertes, analytisch reines Produkt; ^{b} Transmetallierung mit 1,1 Äquiv. CuCN·2LiCl durchgeführt; ^{c} Erhalten mittels Palladium-katalysierter Vernetzung unter Verwendung von Pd(dba)₂ (3 mol %) und (*o*-Furyl)₃P (6 mol %); ^{d} Transmetallierung mit 5 mol % CuCN·2LiCl durchgeführt. | | | | |

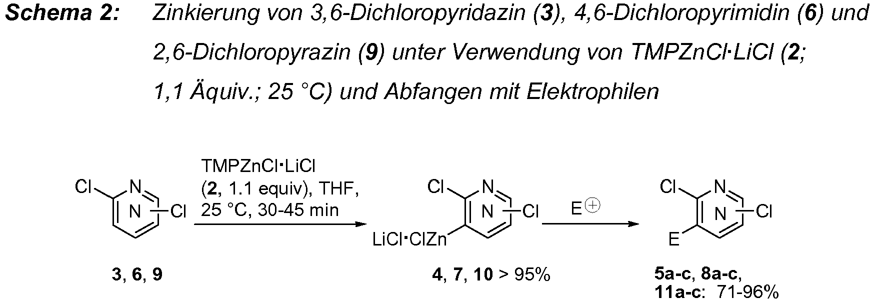

Mehrere empfindliche Heteroarene, wie Pyridazine (Wunderlich, S. H.; Knochel, P. Chem. Comm. 2008, 47, 6387), Pyrimidine ((a) Turck, A.; Plé, N.; Queguiner, G. Heterocycles 1990, 37, 2149. (b) Radinov, R.; Chanev, C.; Haimova, M. J. Org. Chem. 1991, 56, 4793) und Pyrazine (Turck A.; Trohay, D.; Mojovic, L.; Plé, N.; Queguiner, G. J. Organomet. Chem. 1991, 412, 301) werden bei 25 °C unter Verwendung der neuen Base TMPZnCl·LiCl sauber zinkiert **(2;** Schema 2 und Tabelle 1). So führt die Behandlung von 3,6-Dichlorpyridazin **(3)** mit TMPZnCl·LiCl **(2;** 1,1 Äquiv., 25 °C, 30 Minuten) zu der zinkierten Spezies **(4),** die mit I₂, 4-Fluorbenzoylchlorid (nach Transmetallierung mit CuCN·2LiCl) abgefangen werden kann (Knochel, P.; Y-eh, M. C. P.; Berk, S. C.; Talbert, J. J. Org. Chem. 1988, 53, 2390) oder einer Negishi-Kupplung (Negishi, E.; Acc. Chem. Res. 1982, 15, 340) unterworfen werden kann, die zu den erwarteten Produkten **5a-c** in 83-96%igen Ausbeuten führt (Einträge 1-3 von Tabelle 1). Zinkierungen anderer empfindlicher Heteroaromaten können leicht durch Zugabe von TMPZnCl·LiCl **(2)** erzielt werden. So wird das 4,6-Dichlorpyrimidin **(6)** in 45 Minuten bei 25 °C in die 5-zinkierte Spezies umgewandelt. Durch Abfangen mit I₂ wird das lodpyrimidin **8a** in 83%iger Ausbeute bereitgestellt (Eintrag 4). Die Umsetzung mit Furoylchlorid (nach Transmetallierung mit CuCN·2LiCl) (Knochel, P.; Yeh, M. C. P.; Berk, S. C.; Talbert, J. J. Org. Chem. 1988, 53, 2390) liefert das 5-Ketopyrimidin **8b** zu 71% (Eintrag 5). Eine Allylierung (nach Zugabe von CuCN·2LiCl) führt zu dem allylierten Derivat **8c** zu 89% (Eintrag 6). Ebenso wird 2,6-Dichlorpyrazin **(9)** quantitativ mit TMPZnCl·LiCl (**2**; 1,1 Äquiv., 25 °C, 30 Minuten) zinkiert und mit Iod umgesetzt oder einer Negishi-Kupplung (Negishi, E.; Acc. Chem. Res. 1982, 15, 340*)* oder einer Allylierung mit Ethyl-2-(brommethyl)acrylat (Villiéras, J.; Rambaud, M. Org. Synth. 1988, 66, 220) (nach Zugabe von CuCN·2LiCl) unterworfen, was die erwarteten Produkte **11a-c** in 72-90%igen Ausbeuten liefert (Einträge 7-9).

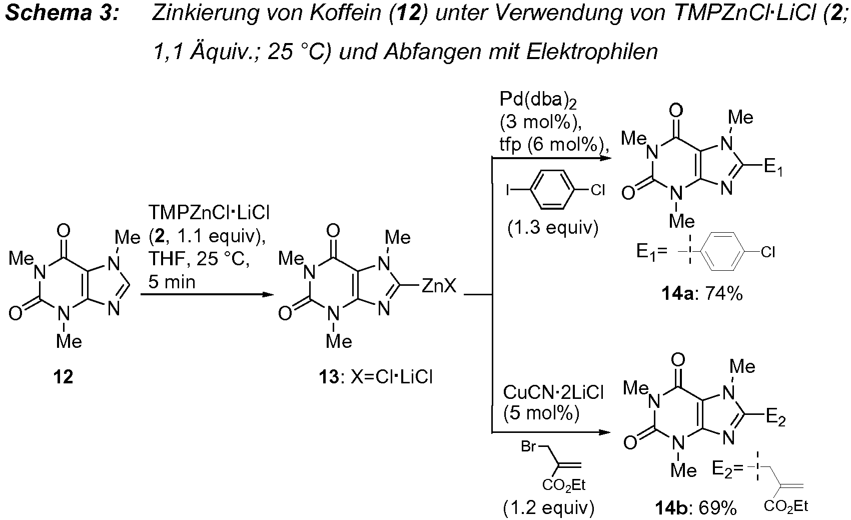

Andere empfindliche Heterozyklen, wie Purine ((a) Boudet, N; Dubbaka, S. R.; Knochel, P. Org. Lett. 2007, 10, 1715. (b) Tobrman, T.; Dvořák, D. Org. Lett. 2006, 8, 1291) können ebenfalls unter milden Bedingungen metalliert werden (Schema 3). So wird Koffein **(12)** (Do, H-Q; Kashif-Khan R. M.; Daugulis, O. J. Am. Chem. Soc. 2008, 130, 15185) einer glatten Zinkierung unter Verwendung von TMPZnCl·LiCl unterworfen (**2**; 1,1 Äquiv., 25 °C, 5 Minuten), was die Zink-Spezies **13** liefert. Eine Negishi-Kupplung (Negishi, E.; Acc. Chem. Res. 1982, 15, 340) oder Abfangen mit Ethyl 2-(brommethyl)acrylat (Villiéras, J.; Rambaud, M. Org. Synth. 1988, 66, 220) (nach Zugabe von CuCN·2LiCl) führt zu dem Purinderivat **14a** und **14b** in Ausbeuten von 74 bzw. 69%.

Ein einzigartiger Vorteil der Zinkbase **2** besteht darin, dass sehr empfindliche funktionelle Gruppen, wie eine Nitrogruppe, bei 25 °C toleriert werden können (I. Sapountzis, P. Knochel, Angew. Chem. Int. Ed. 2002, 41, 1610). So wurde 2,4-Difluornitrobenzol **(15)** in das entsprechende Zinkreagenz **16** durch Behandlung mit TMPZnCl·LiCl (**2**; 1,1 Äquiv., 25 °C, 45 Minuten) umgewandelt. Eine Negishi-Kupplung (Negishi, E.; Acc. Chem. Res. 1982, 15, 340) kann leicht durchgeführt werden, so dass das Arylderivat **17a** in 92%iger Ausbeute (Schema 4) erhalten wird. Einfangen mit Benzoylchlorid (nach Transmetallierung mit CuCN·2LiCl) (Knochel, P.; Yeh, M. C. P.; Berk, S. C.; Talbert, J. J. Org. Chem. 1988, 53, 2390) liefert das Keton **17b** in 84%iger Ausbeute. Nach Abfangen mit I₂ wurde das lodbenzol-Derivat **17c** in 90%iger Ausbeute erhalten.

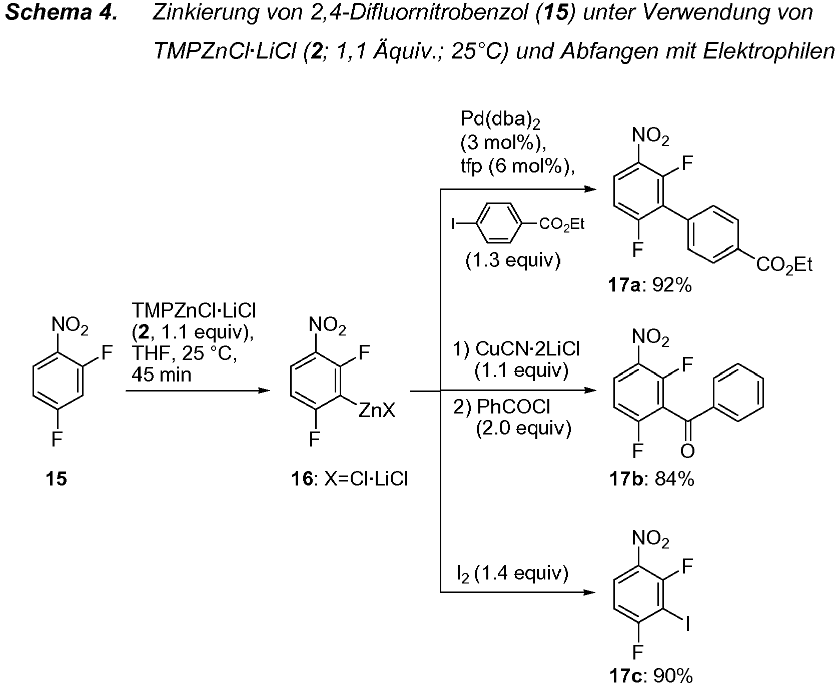

Andere empfindliche elektronenarme Arene und Heteroarene werden ebenfalls unter Verwendung von **2** metalliert. So wird das 2-Chlor-3-nitropyridin **(18)** einer glatten Metallierung mit TMPZnCl·LiCl **(2;** 1,1 Äquiv., 25 °C, 45 Minuten) unterzogen, was die Zink-Spezies **19** liefert. Abfangen unter Verwendung von 3-Bromcyclohexen (nach Zugabe von CuCN·2LiCl) liefert das Pyridin **20** in 73%iger Ausbeute. Ebenso wurde 4-Fluor-1-methoxy-2-nitrobenzol **(21)** in 6 Stunden bei 25 °C in das entsprechende Zinkreagenz **22** umgewandelt. Quenching mit Ethyl-2-(brommethyl)acrylat (Villiéras, J.; Rambaud, M. Org. Synth. 1988, 66, 220) (nach Zugabe von CuCN·2LiCl) führt zu dem allylierten Derivat **23** in 67%iger Ausbeute. Eine Zinkierung von Methyl-5-nitrofuran-2-carboxylat **(24)** kann ebenfalls leicht unter Verwendung von **2** (1,1 Äquiv.) durchgeführt werden und liefert die Zink-Spezies **25** in 30 Minuten bei 25 °C. Eine Allylierung mit 3-Bromcyclohexen (nach Zugabe von CuCN·2LiCl) ergibt das Furan **26** in 72%iger Ausbeute.

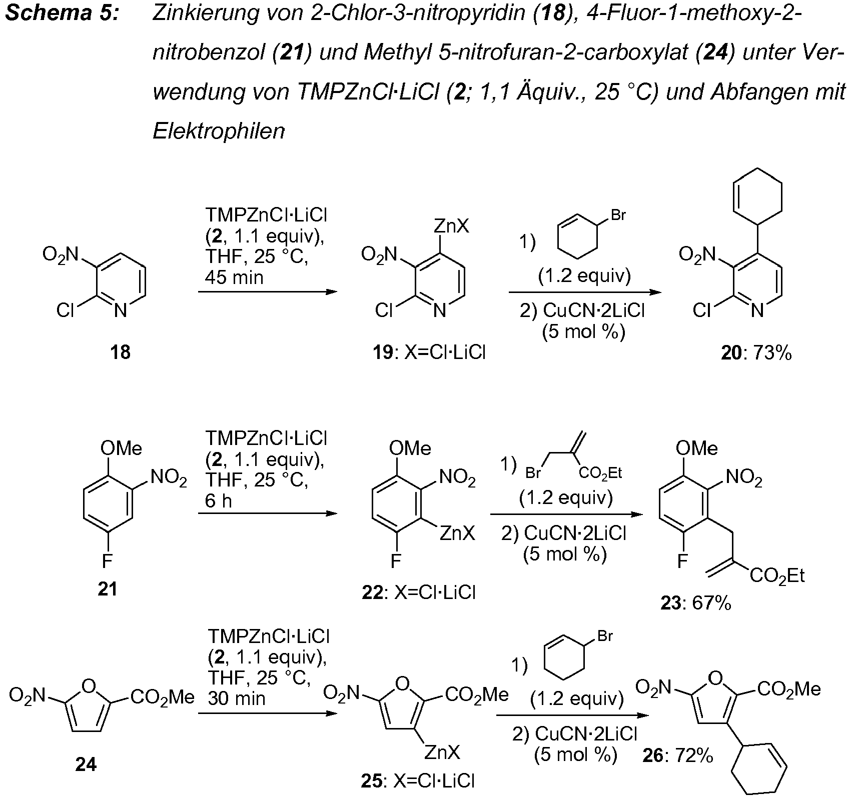

Ein Aldehyd wird ebenfalls gut toleriert ((a) Kneisel, F. F.; Dochnahl, M.; Knochel, P. Angew. Chem. Int. Ed. 2004, 43, 1017. (b) Gong, L.-Z.; Knochel, P. Synlett 2005, 267). So kann der Benzo[*b*]thiophen-3-carbaldehyd **(27)** in die Zink-Spezies **28** bei 25 °C unter Verwendung von TMPZnCl·LiCl (**2;** 1,1 Äquiv.) in 30 Minuten umgewandelt werden. Die Bildung einer anschließenden Kohlenstoff-Kohlenstoff-Bindung wird ebenfalls leicht durch eine Negishi-Kupplung (Negishi, E.; Acc. Chem. Res. 1982, 15, 340) und eine Sonogashira-Reaktion ((a) Benderitter, P.; de Araujo, J. X. Jr.; Schmitt, M.; Bourguignon, J. J. Tetrahedron 2007, 63, 12465. (b) Kim, J. T.; Gevorgyan, V. Org. Lett. 2002, 4, 4697. (c) Sonogashira, K.; Tohda, Y.; Hagihara, N. Tetrahedron Lett. 1975, 50, 4467. (d) Sonogashira, K. Comprehensive Organic Synthesis Pergamon Press: New York, 1991, Bd. 3) durchgeführt, was die Aryl-Heterozyklen **29a-c** in 63-92%iger Ausbeute ergibt.

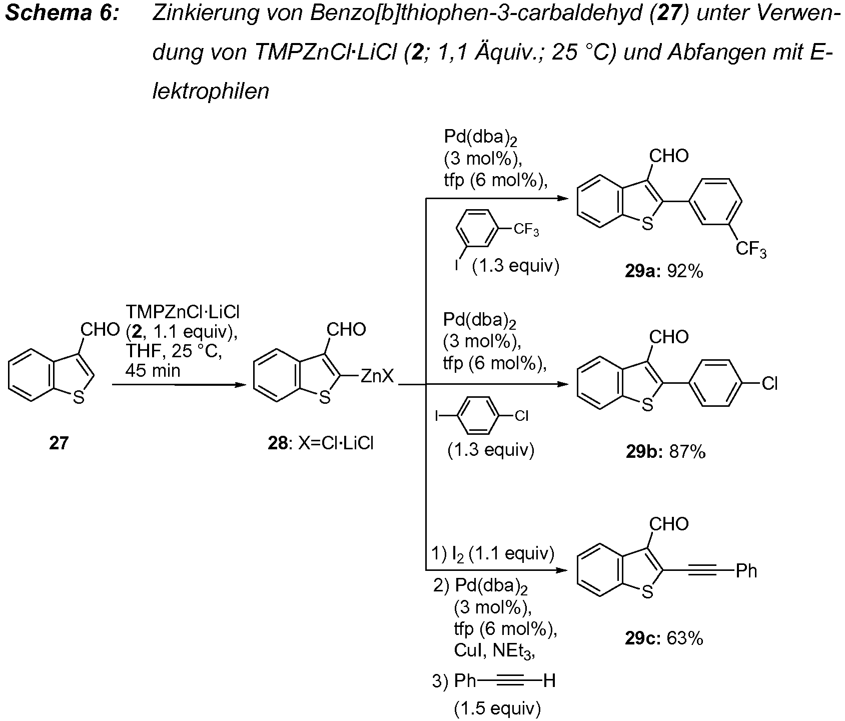

### 2) Experimentelle Verfahren und analytische Daten

### Übliches Verfahren 1: Herstellung des Reagenzes TMPZnCl·LiCl (2):

Ein trockener und mit Argon gespülter 250 ml-Schlenk-Kolben, der mit einem Magnetrührer und einem Septum ausgestattet war, wurde mit frischem 2,2,6,6-Tetramethylpiperidin (10,22 ml, 60 mmol) beschickt, das in THF (60 ml) gelöst war. Diese Lösung wurde auf -40 °C abgekühlt und n-BuLi (2,4 M in Hexan, 25 ml, 60 mmol) wurde tropfenweise zugegeben. Nachdem die Zugabe abgeschlossen war, ließ man das Reaktionsgemisch sich langsam für 1 Stunde auf -10 °C erwärmen. ZnCl₂ (1,0 M in THF, 66 ml, 66 mmol) wurde tropfenweise zugegeben und die so erhaltene Lösung wurde für 30 Minuten bei -10 °C und dann für 30 Minuten bei 25 °C gerührt. Die Lösungsmittel wurden dann unter Vakuum entfernt, wobei ein gelblicher Feststoff zurück blieb. Frisch destilliertes THF wurde dann langsam unter starkem Rühren zugegeben, bis die Salze vollständig gelöst waren. Die frisch hergestellte TMPZnCl·LiCl (**2**)-Lösung wurde vor der Verwendung bei 25 °C mit Benzoesäure unter Verwendung von 4-(Phenylazo)diphenylamin als Indikator titriert. Es wurde eine Konzentration von 1,3 M in THF erhalten.

### Übliches Verfahren für die Zinkierung polyfunktionalisierter Aromaten und Heterozy len mit TMPZnCl·LiCl (TP 2):

Ein trockener und mit Argon gespülter 10 ml-Schlenk-Kolben, der mit einem Magnetrührstab und einem Septum ausgestattet war, wurde mit der Zinkbase (**2**; 1,1 Äquiv.) beschickt. Nach Einstellen der gewünschten Temperatur (Tabelle 1) wurde eine Lösung des entsprechenden Arens (1,0 mmol) in trockenem THF (2 ml) tropfenweise zugegeben und es wurde bei der gleichen Temperatur gerührt. Die Beendigung der Metallierung wurde mittels GC-Analyse von Reaktionsaliquoten überprüft, die mit einer Lösung von I₂ in trockenem THF gequencht wurden.

### Synthese von 3,6-Dichlor-4-iodpyridazin (5a):

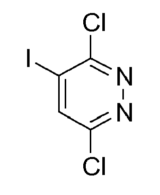

3,6-Dichlorpyridazin (**3**) (149 mg, 1,0 mmol) in THF (2 ml) wurde zu einer Lösung von TMPZnCl·LiCl **(2)** (1,3 M in THF, 0,85 ml, 1,1 mmol) bei 25 °C zugegeben und das Reaktionsgemisch wurde dann bei dieser Temperatur für 30 Minuten gemäß **TP 2** gerührt. In trockenem THF (2 ml) gelöstes I₂ (381 mg, 1,5 mmol) wurde dann tropfenweise hinzugefügt und das so erhaltene Gemisch wurde während 0,5 Stunden gerührt. Das Reaktionsgemisch wurde mit einer gesättigten wässrigen Na₂S₂O₃-Lösung (10 ml) und mit einer gesättigten wässrigen NH₄Cl-Lösung (20 ml) gequencht, mit Diethylether (3 × 50 ml) extrahiert und über wasserfreiem Na₂SO₄ getrocknet. Nach der Filtration wurde das Lösungsmittel unter Vakuum verdampft. Die Reinigung durch Flashchromatographie (CH₂Cl₂/*n*-Pentan, 1:2) lieferte Verbindung **5a** (231 mg, 84%) als farblosen Feststoff. **Schmp.:** 145,1 - 146,6 °C. **¹H**-**NMR (300 MHz, CDCl₃)** *δ*: 8,06 (s, 1 H).
**¹³C-NMR (75 MHz, CDCl₃)** *δ*: 159,7, 153,9, 139,7, 105,4.
**MS (70 eV, EI)** *m*/*z* (%): 274 (95) [M⁺], 127 (23), 123 (10), 121 (10), 119 (100), 86 (15), 84 (43), 49 (8).
**IR (ATR)** *ṽ* (cm⁻¹): 3092, 3020, 1796, 1516, 1488, 1464, 1332, 1296, 1276, 1236, 1152, 1136, 1060, 1044, 992, 956, 900, 812, 764, 728, 672, 660, 628, 608, 588, 564. **HRMS (EI) für C₄HCl₂IN₂** (273,8561): 273,8538.

### Synthese von (3,6-Dichlorpyridazin-4-yl)(4-fluorphenyl)methanon (5b):

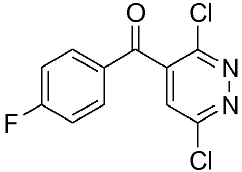

3,6-Dichlorpyridazin (**3**) (149 mg, 1,0 mmol) in THF (2 ml) wurde zu einer Lösung von TMPZnCl*·*LiCl **(2)** (1,3 M in THF, 0,85 ml, 1,1 mmol) bei 25°C zugegeben und das Reaktionsgemisch wurde dann bei dieser Temperatur für 30 Minuten gemäß **TP 2** gerührt. Nach Abkühlen auf -20 °C wurde CuCN·2LiCl (1,0 M in THF, 1,1 mmol, 1,1 Äquiv.) zugegeben und das so erhaltene Gemisch wurde während 30 Minuten bei dieser Temperatur gerührt. 4-Fluorbenzoylchlorid (317 mg, 2,0 mmol) wurde dann langsam zugegeben und man ließ das so erhaltene Gemisch sich langsam auf 10 °C erwärmen. Das Reaktionsgemisch wurde mit einer gesättigten wässrigen NH₄Cl-Lösung (20 ml) gequencht, mit Diethylether (3 × 50 ml) extrahiert und über wasserfreiem Na₂SO₄ getrocknet. Nach der Filtration wurde das Lösungsmittel unter Vakuum verdampft. Die Reinigung durch Flashchromatographie (CH₂Cl₂/*n*-Pentan, 1:1) lieferte Verbindung **5b** (259 mg, 96%) als weißen Feststoff.
**Schmp.:** 71,1 - 72,6 °C.
**¹H-NMR (400 MHz, CDCl₃)** *δ*: 7,79-7,83 (m, 2 H), 7,51 (s, 1 H), 7,19-7,24 (m, 2 H). **¹³C-NMR (100 MHz, CDCl₃)** *δ*: 187,4, 167,0 (d, *J* (C-F) = 259,9 Hz), 156,3, 151,5, 139,6, 132,8 (d, *J* (C-F) = 9,9 Hz), 130,4 (d, *J* (C-F) = 3,1 Hz), 127,7, 116,8 (d, *J* (C-F) = 22,6 Hz).
**MS (70 eV, EI)** *m*/*z* (%): 270 (11) [M⁺], 123 (100), 95 (19).
**IR (ATR)** *ṽ* (cm⁻¹): 3067, 2927, 2358, 1917, 1673, 1590, 1504, 1414, 1344, 1319, 1256, 1237, 1178, 1157, 1140, 1103, 1041, 1009, 967, 955, 909, 849, 841, 818, 795, 760, 753, 683, 659, 650, 645, 638, 633, 625, 620, 614, 606, 602.
**HRMS (EI) für C₁₁H₅Cl₂FN₂O** (269,9763): 269,9762.

### Synthese von 3,6-Dichlor-4-(3-(trifluormethyl)phenyl)-pyridazin (5c):

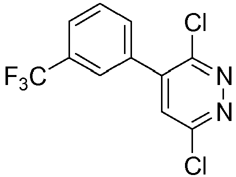

3,6-Dichlorpyridazin (**3**) (149 mg, 1,0 mmol) in THF (2 ml) wurde zu einer Lösung von TMPZnCl*·*LiCl (**2**) (1,3 M in THF, 0,85 ml, 1,1 mmol) bei 25°C zugegeben und das Reaktionsgemisch wurde dann bei dieser Temperatur für 30 Minuten gemäß **TP 2** gerührt. Pd(dba)₂ (17 mg, 3 mol%) und P(o-furyl)₃ (14 mg, 6 mol%), die in THF (2 ml) gelöst und mit 3-Iodbenzomethyltrifluorid (354 mg, 1,3 mmol, 1,3 Äquiv.) gemischt waren, wurden dann mittels einer Kanüle in das Reaktionsgemisch überführt. Das so erhaltene Gemisch wurde während 1 Stunde bei 25 °C gerührt. Das Reaktionsgemisch wurde dann mit einer gesättigten wässrigen NH₄Cl-Lösung (20 ml) gequencht, mit Diethylether (3 × 50 ml) extrahiert und über wasserfreiem Na₂SO₄ getrocknet. Nach der Filtration wurde das Lösungsmittel unter Vakuum verdampft. Die Reinigung durch Flashchromatographie (CH₂Cl₂/*n*-Pentan, 1:2) lieferte Verbindung **5c** (243 mg, 83%) als farblosen Feststoff.
**Schmp.:** 93,0 - 94,9 °C.
**¹H-NMR (400 MHz, CDCl₃)** *δ*: 7,66-7,81 (m, 4 H), 7,53 (s, 1 H).
**¹³C-NMR (100 MHz, CDCl₃)** *δ*: 156,1, 154,4, 143,3, 141,2, 133,9, 131,5 (q, *J* (C-F) = 33,0 Hz), 129,6 (2), 128,3, 127,0 (q, *J* (C-F) = 3,8 Hz), 125,7 (q, *J* (C-F) = 3,8 Hz), 123,4 (q, *J* (C-F) = 272,5 Hz).
**MS (70 eV, EI)** *m*/*z* (%): 294 (60), 292 (100) [M⁺], 266 (17), 264 (25), 229 (28), 206 (16), 204 (49), 194 (21), 169 (13), 138 (10), 136 (24), 113 (25), 59 (18).
**IR (ATR)** *ṽ* (cm⁻¹): 3048, 2359, 1743, 1614, 1558, 1485, 1435, 1361, 1323, 1309, 1281, 1241, 1226, 1214, 1167, 1144, 1109, 1097, 1078, 1060, 1042, 1001, 933, 917, 903, 884, 803, 782, 755, 709, 697, 660, 645, 639, 632, 625, 620, 614, 606, 601. **HRMS (EI) für C₁₁H₅Cl₂F₃N₂** (291.9782): 291.9785.

### Synthese von 4,6-Dichlor-5-iodpyrimidin (8a):

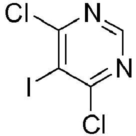

4,6-Dichlorpyrimidin **6** (149 mg, 1,0 mmol) in THF (2 ml) wurde zu einer Lösung von TMPZnCl·LiCl **(2)** (1,3 M in THF, 0,85 ml, 1,1 mmol) bei 25 °C zugegeben und das Reaktionsgemisch wurde dann bei dieser Temperatur für 45 Minuten gemäß **TP 2** gerührt. In trockenem THF (2 ml) gelöstes I₂ (381 mg, 1,5 mmol) wurde dann tropfenweise hinzugefügt und das so erhaltene Gemisch wurde während 0,5 Stunden gerührt. Das Reaktionsgemisch wurde mit einer gesättigten wässrigen Na₂S₂O₃-Lösung (10 ml) und mit einer gesättigten wässrigen NH₄Cl-Lösung (20 ml) gequencht, mit Diethylether (3 × 50 ml) extrahiert und über wasserfreiem Na₂SO₄ getrocknet. Nach der Filtration wurde das Lösungsmittel unter Vakuum verdampft. Die Reinigung durch Flashchromatographie (CH₂Cl₂/*n*-Pentan, 1:4) lieferte Verbindung **8a** (227 mg, 83%) als farblosen Feststoff.
**Schmp**.:134,9-136,5°C.
**¹H NMR (300 MHz, CDCl₃)** *δ*: 8,65 (s, 1 H).
**¹³C NMR (75 MHz, CDCl₃)** *δ*: 166,6, 156,8, 98,9.
MS (70 eV, **EI)** *m*/*z* (%): 274 (100) [M⁺], 239 (27), 97 (12), 83 (12), 57 (21).
**IR (ATR)** *ṽ* (cm⁻¹): 2923, 2855, 1900, 1499, 1386, 11341, 1296, 1214, 1080, 1014, 790,763,745.
**HRMS (EI) für C₄HCl₂IN₂** (273,8561): 273,8565.

### Synthese von (4,6-Dichlorpyrimidin-5-yl)(furan-2-yl)methanon (8b):

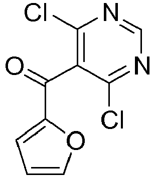

4,6-Dichlorpyrimidin **6** (149 mg, 1,0 mmol) in THF (2 ml) wurde zu einer Lösung von TMPZnCl·LiCl **(2)** (1,3 M in THF, 0,85 ml, 1,1 mmol) bei 25 °C zugegeben und das Reaktionsgemisch wurde dann bei dieser Temperatur für 45 Minuten gemäß **TP 2** gerührt. CuCN·2LiCl (1,0 M Lösung in THF, 1,1 ml, 1,1 mmol) wurde langsam bei -20 °C hinzugefügt und das Reaktionsgemisch wurde bei der gleichen Temperatur während 30 Minuten gerührt. Dann wurde Furan-2-carbonylchlorid (261 mg, 2,0 mmol) tropfenweise bei -20 °C zugegeben und man ließ das so erhaltene Gemisch sich langsam über Nacht auf 25 °C erwärmen. Das Reaktionsgemisch wurde mit einer gesättigten wässrigen NH₄Cl-Lösung (30 ml) gequencht, mit Diethylether (5 × 30 ml) extrahiert und über wasserfreiem Na₂SO₄ getrocknet. Nach der Filtration wurde das Lösungsmittel unter Vakuum verdampft. Die Reinigung durch Flashchromatographie (CH₂Cl₂/*n*-Pentan 1:1) lieferte **8b** als farblosen Feststoff (172 mg, 71%).
**Schmp.:** 143,6 - 145,4 °C.
**¹H NMR (400 MHz, CDCl₃)** *δ*: 8,88 (s, 1 H), 7,70 (m, 1 H), 7,28 (m, 1 H), 6,66 (m, 1 H).
**¹³C NMR (100 MHz, CDCl₃)** *δ*: 175,6, 158,8, 158,4, 150,8, 149,0, 130,9, 121,5, 113,5. **MS (70 eV, EI)** *m*/*z* (%): 242 (48) [M⁺], 167 (49), 95 (100), 58 (21), 43 (33).
**IR (ATR)** *ṽ* (cm⁻¹): 3133, 2969, 2359, 2340, 1738, 1636, 1558, 1540, 1512, 1450, 1403, 1375, 1361, 1297, 1230, 1216, 1168, 1123, 1083, 1032, 956, 904, 888, 878, 815,789,781,746,738,668,626,615,609.
**HRMS (EI)** für **C₉H₄Cl₂N₂O₂** (241,9650): 241,9653.

### Synthese von 5-Allyl-4,6-dichlorpyrimidin (8c):

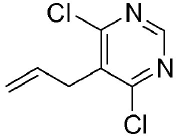

4,6-Dichlorpyrimidin **6** (149 mg, 1,0 mmol) in THF (2 ml) wurde zu einer Lösung von TMPZnCl·LiCl (**2**) (1,3 M in THF, 0,85 ml, 1,1 mmol) bei 25 °C zugegeben und das Reaktionsgemisch wurde dann bei dieser Temperatur für 45 Minuten gemäß **TP 2** gerührt. CuCN·2LiCl (1 M in THF; 0,05 ml, 5 mol %) wurde dann langsam bei -20 °C zugegeben. Allylbromid (242 mg, 2,0 mmol) wurde dann langsam bei -60 °C zugegeben. Man ließ das so erhaltene Gemisch sich dann langsam während 4 Stunden bis auf 0 °C erwärmen. Das Reaktionsgemisch wurde mit einer gesättigten wässrigen NH₄Cl-Lösung (20 ml) gequencht, mit Diethylether (5 × 30 ml) extrahiert und über wasserfreiem Na₂SO₄ getrocknet. Nach der Filtration wurde das Lösungsmittel unter Vakuum verdampft. Die Reinigung durch Flashchromatographie (CH₂Cl₂/*n*-Pentan 1:2) lieferte **8c** als farblosen Feststoff (215 mg, 89%).
**¹H NMR (300 MHz, CDCl₃)** *δ*: 8,64 (s, 1 H), 5,80-5,90 (m, 1 H), 5,09-5,18 (m, 2 H), 3,64 (dt, ³ *J* = 6,4 Hz, ⁴ *J =* 1,4 Hz, 2 H).
**¹³C NMR (75 MHz, CDCl₃)** *δ*: 162,0, 155,8, 130,9, 130,6, 118,2, 34,0.
**MS (70 eV, EI)** *m*/*z* (%): 188 (70) [M⁺], 125 (22), 117 (44), 90 (59), 64 (35), 49 (43), 41 (100).
**IR (ATR)** *ṽ* (cm⁻¹): 2969, 2360, 1739, 1639, 1539, 1513, 1435, 1406, 1375, 1348, 1313, 1290, 1200, 1162, 1129, 1090, 989, 929, 906, 839, 777, 687, 668, 627, 621, 616.
**HRMS (EI)** für **C₇H₆Cl₂N₂** (187,9908): 187,9913.

### Synthese von 3,5-Dichlor-2-iodpyrazin (11a):

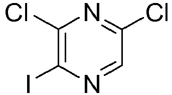

2,6-Dichlorpyrazin **(9)** (149 mg, 1,0 mmol) in THF (2 ml) wurde zu einer Lösung von TMPZnCl·LiCl **(2)** (1,3 M in THF, 0,85 ml, 1,1 mmol) bei 25 °C zugegeben und das Reaktionsgemisch wurde dann bei dieser Temperatur für 30 Minuten gemäß **TP 2** gerührt. In trockenem THF (2 ml) gelöstes I₂ (381 mg, 1,5 mmol) wurde dann tropfenweise hinzugefügt und das so erhaltene Gemisch wurde während 0,5 Stunden gerührt. Das Reaktionsgemisch wurde mit einer gesättigten wässrigen Na₂S₂O₃-Lösung (10 ml) und mit einer gesättigten wässrigen NH₄Cl-Lösung (20 ml) gequencht, mit Diethylether (3 × 50 ml) extrahiert und über wasserfreiem Na₂SO₄ getrocknet. Nach der Filtration wurde das Lösungsmittel unter Vakuum verdampft. Die Reinigung durch Flashchromatographie (CH₂Cl₂/*n*-Pentan, 1:2) lieferte Verbindung **11a** (251 mg, 90%) als farblosen Feststoff.
**Schmp.:** 101,3 - 103,0 °C.
**¹H**-**NMR (300 MHz, CDCl₃)** *δ*: 8,30 (s, 1 H).
**¹³C-NMR (75 MHz, CDCl₃)** *δ*: 153,1, 146,9, 142,4, 115,7.
**MS (70 eV, EI)** *m*/*z* (%): 274 (100) [M⁺], 147 (75), 127 (18), 86 (32), 57 (21), 44 (94). **IR (ATR)** *ṽ* (cm⁻¹): 2969, 2633, 2281, 1784, 1738, 1510, 1491, 1379, 1353, 1323, 1274, 1230, 1217, 1205, 1175, 1162, 1143, 1018, 893, 843, 655, 634, 618, 611, 604. **HRMS (EI) für C₄HCl₂IN₂** (273,8561): 273,8555.

### Synthese von Ethyl-4-(3,5-dichlorpyrazin-2-yl)benzoat (11b):

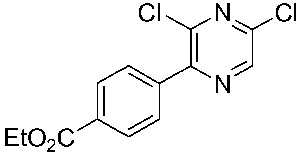

2,6-Dichlorpyrazin **(9)** (149 mg, 1,0 mmol) in THF (2 ml) wurde zu einer Lösung von TMPZnCl·LiCl (**2**) (1,3 M in THF, 0,85 ml, 1,1 mmol) bei 25 °C zugegeben und das Reaktionsgemisch wurde dann bei dieser Temperatur während 30 Minuten gemäß **TP 2** gerührt. Pd(dba)₂ (17 mg, 3 mol%) und P(*o*-furyl)₃ (14 mg, 6 mol%), gelöst in THF (2 ml), wozu anschließend Ethyl-4-iodbenzoat (359 mg, 1,3 mmol) hinzugefügt wurde, wurden dann mittels einer Kanüle in das Reaktionsgemisch überführt. Das Reaktionsgemisch wurde bei 25 °C während 1,5 Stunden mit einer gesättigten wässrigen NH₄Cl-Lösung (20 ml) gerührt, mit Diethylether (3 × 50 ml) extrahiert und über wasserfreiem Na₂SO₄ getrocknet. Nach der Filtration wurde das Lösungsmittel unter Vakuum verdampft. Die Reinigung durch Flashchromatographie (CH₂Cl₂/*n*-Pentan, 1:2) lieferte Verbindung **11 b** (251 mg, 87%) als farblosen Feststoff. **Schmp.:** 88,5 - 90,0 °C.
**¹H-NMR (300 MHz, CDCl₃)** *δ*: 8,59 (s, 1 H), 8,14 (d, *J* = 8,6 Hz, 2 H), 7,84 (d, *J* = 8,6 Hz, 2 H), 4,40 (q, *J* = 7,2 Hz, 2 H), 1,40 (t, *J* = 7,0 Hz, 3 H).
**¹³C-NMR (75 MHz, CDCl₃)** *δ*: 165,8, 150,1, 145,9, 142,0, 139,0, 131,6, 129,4 (2), 61,2, 14,3.
**MS (70 eV, EI)** *m*/*z* (%): 296 (32) [M⁺], 270 (24), 268 (38), 251 (100), 223 (26).
**IR (ATR)** *ṽ* (cm⁻¹): 3086, 3005, 2985, 2359, 1966, 1708, 1611, 1569, 1537, 1507, 1482, 1466, 1446, 1423, 1408, 1366, 1310, 1283, 1263, 1190, 1175, 1140, 1131, 1114, 1098, 1028, 1021, 1009, 915, 858, 843, 786, 758, 719, 698, 657, 634, 621, 616, 610,602.
**HRMS (EI) für C₁₃H₁₀Cl₂N₂O₂** (296,0119): 296,0119.

### Synthese von Ethyl-2-((3,5-dichlorpyrazin-2-yl)methyl)acrylat (11c):

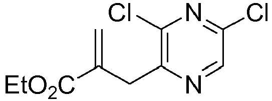

2,6-Dichlorpyrazin **(9)** (149 mg, 1,0 mmol) in THF (2 ml) wurde zu einer Lösung von TMPZnCl·LiCl (**2**) (1,3 M in THF, 0,85 ml, 1,1 mmol) bei 25 °C zugegeben und das Reaktionsgemisch wurde dann bei dieser Temperatur während 30 Minuten gemäß **TP 2** gerührt. Nach Abkühlen auf -50 °C wurden Ethyl-2-(brommethyl)acrylat (230 mg, 1,2 mmol) und CuCN·2LiCl (1,0 M Lösung in THF, 5 Tropfen) hinzugefügt und man ließ das so erhaltene Gemisch sich langsam auf -20 °C erwärmen. Das Reaktionsgemisch wurde mit einer gesättigten wässrigen NH₄Cl-Lösung (20 ml) gequencht, mit Diethylether (3 × 50 ml) extrahiert und über wasserfreiem Na₂SO₄ getrocknet. Nach der Filtration wurde das Lösungsmittel unter Vakuum verdampft. Die Reinigung durch Flashchromatographie (CH₂Cl₂/*n*-Pentan, 1:3) lieferte Verbindung **11c** (187 mg, 72%) als farbloses Öl.
**¹H-NMR (300 MHz, CDCl₃)** *δ*: 8,38 (s, 1 H), 6,34 (s, 1 H), 5,56 (s, 1 H), 4,14 (q, *J* = 7,1 Hz, 2 H), 3,92 (s, 2 H), 1,21 (t, *J* = 7,1 Hz, 3 H).
**¹³C-NMR (75 MHz, CDCl₃)** *δ*: 166,0, 151,5, 146,8, 145,0, 141,5, 136,0, 127,6, 60,9, 36,7, 14,0.
**MS (70 eV, EI)** *m*/*z* (%): 261 (100) [M⁺-H], 163 (10).
**IR (ATR)** *ṽ* (cm⁻¹): 2969, 2359, 1738, 1503, 1385, 1342, 1294, 1226, 1215, 1084, 1013, 987, 954, 795, 764, 749, 667, 621, 615, 608, 603.
**HRMS (ESI) für C₁₀H₁₀Cl₂N₂O₂** (260,0119 (M⁺-H)): 261,0196.

### Synthese von 8-(4-Chlorphenyl)-1,3,7-trimethyl-1H-purin-2,6(3H,7H)-dion (14a):

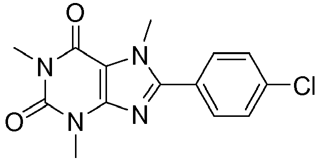

TMPZnCl·LiCl (**2**) (1,3 M in THF, 0.85 ml, 1,1 mmol) wurde zu einer Lösung von 1,3,7-Trimethyl-1 H-purin-2,6(3H,7H)-dion **(12)** (194 mg, 1,0 mmol) in THF (2 ml) bei 25 °C zugegeben und das Reaktionsgemisch wurde dann bei dieser Temperatur während maximal 5 Minuten gerührt. Pd(dba)₂ (17 mg, 3 mol%) und P(ofuryl)₃ (14 mg, 6 mol%), gelöst in THF (2 ml) und mit 1-Chlor-4-iodbenzol (310 mg, 1,3 mmol, 1,3 Äquiv.) gemischt, wurden dann mittels einer Kanüle in das Reaktionsgemisch überführt. Das so erhaltene Gemisch wurde während 1 Stunde bei 25 °C gerührt. Das Reaktionsgemisch wurde dann mit einer gesättigten wässrigen NH₄Cl-Lösung (20 ml) gequencht, mit Diethylether (3 × 50 ml) extrahiert und über wasserfreiem Na₂SO₄ getrocknet. Nach der Filtration wurde das Lösungsmittel unter Vakuum verdampft. Die Reinigung durch Flashchromatographie (CH₂Cl₂/Ether, 1:1) lieferte Verbindung **14a** (226 mg, 74%) als farblosen Feststoff.
**¹H-NMR (300 MHz, CDCl₃)** *δ*: 7,62 (d, *J* = 8,5 Hz, 2 H), 7,48 (d, *J* = 8,5 Hz, 2 H), 4,03 (s, 3 H), 3,59 (s, 3 H), 3,39 (s, 3 H).
**¹³C-NMR (75 MHz, CDCl₃)** *δ*: 155,4, 151,5, 150,7, 148,1, 136,7, 130,4, 129,2, 126,7, 108,6, 33,9, 29,8, 28,0.
**MS (70 eV, EI)** *m*/*z* (%): 304 (100) [M⁺], 82 (23), 67 (13).
**IR (ATR)** *ṽ* (cm⁻¹): 2969, 1738, 1694, 1646, 1605, 1569, 1538, 1473, 1454, 1430, 1408, 1374, 1288, 1229, 1216, 1180, 1108, 1090, 1074, 1030, 1008, 977, 835, 803, 759, 749, 739, 730, 708, 685, 671, 650, 645, 639, 632, 625, 620, 614, 606. 601. **HRMS (ESI) für C₁₄H₁₃ClN₄O₂** (304,0727): 304,0722.

### Synthese von Ethyl-2-((1,3,7-trimethyl-2,6-dioxo-2,3,6,7-tetrahydro-1H-purin-8-yl)-methyl)acrylat (14b):

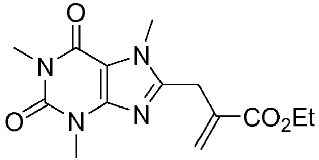

TMPZnCl·LiCl (**2**) (1,3 M in THF, 0,85 ml, 1,1 mmol) wurde zu einer Lösung von 1,3,7-Trimethyl-1 H-purin-2,6(3H,7H)-dion **(12)** (194 mg, 1,0 mmol) in THF (2 ml) bei 25 °C zugegeben und das Reaktionsgemisch wurde dann bei dieser Temperatur während maximal 5 Minuten gerührt. Nach Abkühlen auf -50 °C wurden Ethyl-2-(brommethyl)acrylat (230 mg, 1,2 mmol) und CuCN·2LiCl (1,0 M Lösung in THF, 5 Tropfen) hinzugefügt und man ließ das so erhaltene Gemisch sich langsam über Nacht erwärmen. Das Reaktionsgemisch wurde mit einer gesättigten wässrigen NH₄Cl-Lösung (20 ml) gequencht, mit Diethylether (3 × 50 ml) extrahiert und über wasserfreiem Na₂SO₄ getrocknet. Nach der Filtration wurde das Lösungsmittel unter Vakuum verdampft. Die Reinigung durch Flashchromatographie (CH₂Cl₂/Ether, 1:1) lieferte Verbindung **14b** (211 mg, 69%) als farblosen Feststoff.
**¹H-NMR (300 MHz, CDCl₃)** *δ*: 6,28 (s, 1 H), 5,49 (s, 1 H), 4,14 (q, *J* = 7,1 Hz, 2 H), 3,86 (s, 3 H), 3,70 (s, 2 H), 3,45 (s, 3 H), 3,29 (s, 3 H), 1,21 (t, *J* = 7,1 Hz, 3 H).**¹³C-NMR (75 MHz, CDCl₃)** *δ*: 165,7, 155,1, 151,4, 150,8, 147,7, 135,0, 127,3, 107,4, 61,1, 31,8, 29,6, 29,3, 27,7, 14,0.
**MS (70 eV, EI)** *m*/*z* (%): 306 (78) [M⁺], 260 (28), 232 (100), 219 (11), 67 (13).
**IR (ATR)** *ṽ* (cm⁻¹): 2998, 2956, 2358, 1719, 1697, 1658, 1548, 1497, 1448, 1426, 1402, 1362, 1340, 1293, 1253, 1215, 1162, 1112, 1033, 978, 960, 939, 894, 858, 831, 812,759,743,718,693,663,641,630,602.
**HRMS (ESI) für C₁₄H₁₈N₄O₄** (306,1328): 306,1320.

### Synthese von Ethyl-2',6'-difluor-3'-nitrobiphenyl-4-carboxylat (17a):

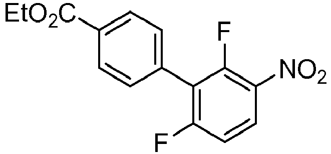

2,4-Difluor-1-nitrobenzol **15** (159 mg, 1,0 mmol) in THF (2 ml) wurde zu einer Lösung von TMPZnCl·LiCl (**2**) (1,3 M in THF, 0,85 ml, 1,1 mmol) bei 25 °C zugegeben und das Reaktionsgemisch wurde dann bei dieser Temperatur während 45 Minuten gemäß **TP 2** gerührt. Pd(dba)₂ (17 mg, 3 mol%) und P(*o*-furyl)₃ (14 mg, 6 mol%), gelöst in THF (2 ml), zu dem anschließend Ethyl-4-iodbenzoat (359 g, 1,3 mmol) hinzugefügt wurde, wurden dann mittels einer Kanüle bei -20°C überführt. Das so erhaltene Gemisch ließ man sich langsam über Nacht auf 25 °C erwärmen. Das Reaktionsgemisch wurde dann mit einer gesättigten wässrigen NH₄Cl-Lösung (20 ml) gestoppt, mit Diethylether (3 × 50 ml) extrahiert und über wasserfreiem Na₂SO₄ getrocknet. Nach der Filtration wurde das Lösungsmittel unter Vakuum verdampft. Die Reinigung durch Flashchromatographie (CH₂Cl₂/*n*-Pentan, 1:2) lieferte Verbindung **17a** (281 mg, 92%) als farblosen Feststoff.
**Schmp.:** 85,0 - 86,7 °C.
**¹H NMR (300 MHz, CDCl₃)** *δ*: 8,09-8,18 (m, 1 H), 8,15 (d, *J =* 8,8 Hz, 2 H), 7,51 (d, *J* = 8,8 Hz, 2 H), 7,11-7,18 (m, 1 H), 4,40 (q, *J* = 7,0 Hz, 3 H), 1,40 (d, *J* = 7,0 Hz, 2 H). **¹³C NMR (75 MHz, CDCl₃)** *δ*: 165,8, 162,5 (dd, *J* = 6,0 Hz, *J* = 260,1 Hz), 153,7 (dd, *J* = 6,0 Hz, *J =* 260,1 Hz), 131,2 (dd, *J* = 0,5 Hz, *J* = 3,9 Hz), 130,2 (dd, *J* = 1,8 Hz, *J =* 2,0 Hz), 129,7, 126,6 (dd, *J* = 1,8 Hz, *J* = 21,4 Hz), 120,2 (dd, *J* = 28,1 Hz, *J* = 1,8 Hz), 112,1 (dd, *J* = 4,3 Hz, *J* = 24,7 Hz), 61,3, 14,3.
**MS (70 eV, EI)** *m*/*z* (%): 307 (23) [M⁺], 279 (48), 262 (100), 216 (43), 188 (34), 44 (12).
**IR (ATR)** *ṽ* (cm⁻¹): 3101, 2969, 2359, 1712, 1621, 1589, 1567, 1535, 1510, 1472, 1404, 1368, 1341, 1304, 1286, 1269, 1215, 1185, 1170, 1148, 1127, 1103, 1070, 1020, 1011, 948, 879, 857, 824, 778, 756, 714, 702, 667, 636, 620, 607, 602.
**HRMS (EI) für C₁₅H₁₁F₂NO₄** (307,0656): 307,0651.

### Synthese von (2,6-Difluor-3-nitrophenyl)(phenyl)methanon (17b):

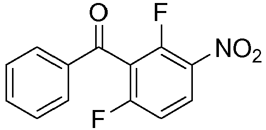

2,4-Difluor-1-nitrobenzol **15** (159 mg, 1,0 mmol) in THF (2 ml) wurde zu einer Lösung von TMPZnCl·LiCl (**2**) (1,3 M in THF, 0,85 ml, 1,1 mmol) bei 25 °C zugegeben und das Reaktionsgemisch wurde dann bei dieser Temperatur während 45 Minuten gemäß **TP 2** gerührt. CuCN·2LiCl (1,0 M Lösung in THF, 1,1 ml, 1,1 mmol) wurde langsam bei -40 °C hinzugefügt und das Reaktionsgemisch wurde bei der gleichen Temperatur während 30 Minuten gerührt. Dann wurde Benzoylchlorid (281 mg, 2,0 mmol) tropfenweise bei -40 °C zugegeben und das so erhaltene Gemisch ließ man sich langsam über Nacht auf 25 °C erwärmen. Das Reaktionsgemisch wurde dann mit einer gesättigten wässrigen NH₄Cl-Lösung (20 ml) gequencht, mit Diethylether (3 × 50 ml) extrahiert und über wasserfreiem Na₂SO₄ getrocknet. Nach der Filtration wurde das Lösungsmittel unter Vakuum verdampft. Die Reinigung durch Flashchromatographie (CH₂Cl₂/*n*-Pentan, 1:2) lieferte Verbindung 17b (221 mg, 84%) als farblosen Feststoff.
**Schmp.:** 75,8 - 77,2 °C.
**¹H NMR (300 MHz, CDCl₃)** *δ*: 7,14-8,31 (m, 7 H).
**¹³C NMR (75 MHz, CDCl₃)** *δ*: 186,2, 162,2 (dd, *J* = 4,2 Hz, *J* = 262,4 Hz), 153,7 (dd, *J* = 9,0 Hz, *J* = 269,9 Hz), 135,7, 135,1, 133,8, 130,2, 129,6, 129,1, 128,7 (dd, *J* = 2,1 Hz, *J =* 0,9 Hz), 128,5, 119,3 (dd, *J* = 21,9 Hz, *J* = 2,1 Hz).
**MS (70 eV, EI)** *m*/*z* (%): 263 (52) [M⁺], 105 (100), 33 (77).
**IR (ATR)** *ṽ* (cm⁻¹): 3100, 1912, 1738, 1675, 1619, 1594, 1530, 1496, 1469, 1450, 1351, 1320, 1311, 1280, 1266, 1217, 1180, 1159, 1128, 1100, 1073, 1034, 1027, 1000, 970, 934, 862, 834, 828, 797, 774, 759, 731, 705, 692, 683, 668, 645, 638, 630, 626,620,614,606,601.
**HRMS (EI) für C₁₃H₇F₂NO₃** (263,0394): 263,0393.

### Synthese von 1,3-Difluor-2-iod-4-nitrobenzol (17c):

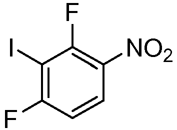

2,4-Difluor-1-nitrobenzol **15** (159 mg, 1,0 mmol) in THF (2 ml) wurde zu einer Lösung von TMPZnCl·LiCl (**2**) (1,3 M in THF, 0,85 ml, 1,1 mmol) bei 25 °C zugegeben und das Reaktionsgemisch wurde dann bei dieser Temperatur während 45 Minuten gemäß **TP 2** gerührt. In trockenem THF (2 ml) gelöstes I₂ (381 mg, 1,5 mmol) wurde dann tropfenweise hinzugefügt und das so erhaltene Gemisch wurde während 0,5 Stunden gerührt. Das Reaktionsgemisch wurde mit einer gesättigten wässrigen Na₂S₂O₃-Lösung (10 ml) und mit einer gesättigten wässrigen NH₄Cl-Lösung (20 ml) gequencht, mit Diethylether (3 × 50 ml) extrahiert und über wasserfreiem Na₂SO₄ getrocknet. Nach der Filtration wurde das Lösungsmittel unter Vakuum verdampft. Die Reinigung durch Flashchromatographie (CH₂Cl₂/*n*-Pentan, 1:1) lieferte Verbindung **17c** (256 mg, 90%) als farblosen Feststoff.
**Schmp.:** 46,1 - 47,5 °C.
**¹H NMR (300 MHz, CDCl₃)** *δ*: 8,12-8,17 (m, 1 H), 7,04-7,08 (m, 1 H).
**¹³C NMR (75 MHz, CDCl₃)** *δ*: 165,6 (dd, *J* = 5,0 Hz, *J* = 252,6 Hz), 156,4 (dd, *J* = 6,9 Hz, *J* = 264,1 Hz), 127,7 (dd, *J* = 2,3 Hz, *J =* 10,3 Hz), 111,6 (dd, *J* = 4,2 Hz, *J =* 26,1 Hz), 74,3 (dd, *J* = 29,2 Hz, *J* = 1,9 Hz).
**MS (70 eV, EI)** *m*/*z* (%): 285 (100) [M⁺], 258 (17), 239 (19), 227 (17), 167 (25), 149 (66), 112 (58), 71 (11), 57 (12), 44 (12).
**IR (ATR)** *ṽ* (cm⁻¹): 3098, 2926, 2855, 2359, 1916, 1739, 1602, 1584, 1529, 1463, 1425, 1336, 1301, 1277, 1218, 1147, 1105, 1011, 860, 827, 751, 698, 669, 621, 616. **HRMS (EI) für C₆H₂F₂INO₂** (284,9098): 284,9094.

### Synthese von 2-Chlor-4-cyclohex-2-enyl-3-nitropyridin (20):

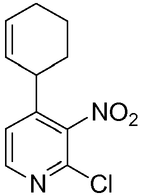

2-Chlor-3-nitropyridin (**18**) (159 mg, 1,0 mmol) in THF (2 ml) wurde zu einer Lösung von TMPZnCl·LiCl (**2**) (1,3 M in THF, 0,85 ml, 1,1 mmol) bei 25 °C zugegeben und das Reaktionsgemisch wurde dann bei dieser Temperatur während 30 Minuten gemäß **TP 2** gerührt. Nach Abkühlen auf -50 °C wurden 3-Bromcyclohexen (192 mg, 1,2 mmol) und CuCN·2LiCl (1,0 M Lösung in THF, 0,05 ml, 0,05 mmol) hinzugefügt und das Reaktionsgemisch wurde während 1 Stunde bei der gleichen Temperatur gerührt. Das Reaktionsgemisch wurde mit einer gesättigten wässrigen NH₄Cl-Lösung (20 ml) gequencht, mit Diethylether (3 × 50 ml) extrahiert und über wasserfreiem Na₂SO₄ getrocknet. Nach der Filtration wurde das Lösungsmittel unter Vakuum verdampft. Die Reinigung durch Flashchromatographie (CH₂Cl₂/*n*-Pentan, 1:1) lieferte 2-Chlor-4-cyclohex-2-enyl-3-nitropyridin (**20**) (173 mg, 73%) als farblosen Feststoff. **Schmp.:** 54,5 - 55,4 °C.
**¹H-NMR (300 MHz, CDCl₃)** *δ*: 8,44 (d, ³*J* = 5,1 Hz, 1 H), 7,32 (d, ³*J* = 5,1 Hz, 1 H), 6,07 (ddd, ³*J* = 10,0 Hz, ³*J* = 6,1 Hz, ⁴*J* = 3,7 Hz, 1 H), 5,54 (dd, ³*J* = 10,0, ⁴*J =* 1,9 Hz, 1 H), 3,46 (m, 1 H), 2,09 (m, 3 H), 1,76 (m, 1 H), 1,64 (m, 1 H), 1,51 (m, 1 H). **¹³C-NMR (75 MHz, CDCl₃)** *δ*: 150,2, 150,0, 146,5, 141,8, 131,9, 125,9, 123,3, 37,4, 31,3, 24,7, 20,8.
**MS (70 eV, EI)** *m*/*z* (%): 237 (3) [M⁺-H], 223 (31), 221 (100), 203 (48), 193 (48), 185 (20), 181 (45), 167 (32), 165 (31), 157 (21), 129 (29), 128 (31), 115 (21), 77 (35), 51 (22), 41 (34).
**IR (ATR)** *ṽ* (cm⁻¹): 2939, 1589, 1539, 1446, 1361, 1347, 1231, 1137, 1041, 973, 918, 890,855,845,757,723,691,616.
**HRMS (EI) für C₁₁H₁₁ClN₂O₂** (237,0431 [M⁺-H]): 237,0424 [M⁺-H].

### Synthese von Ethyl-2-(6-fluor-3-methoxy-2-nitrobenzyl)acrylat (23):

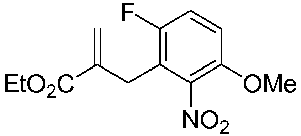

4-Fluor-1-methoxy-2-nitrobenzol **(21)** (171 mg, 1,0 mmol) in THF (2 ml) wurde zu einer Lösung von TMPZnCl·LiCl (**2**) (1,3 M in THF, 0,85 ml, 1,1 mmol) bei 25 °C zugegeben und das Reaktionsgemisch wurde bei dieser Temperatur während 6 Stunden gemäß **TP 2** gerührt. Nach Abkühlen auf -50 °C wurden Ethyl-2-(brommethyl)acrylat (230 mg, 1,2 mmol) und CuCN·2LiCl (1,0 M Lösung in THF, 5 Tropfen) bei -40 °C hinzugefügt und das so erhaltene Gemisch wurde bei der gleichen Temperatur während 1 Stunde gerührt. Das Reaktionsgemisch wurde mit einer gesättigten wässrigen NH₄Cl-Lösung (20 ml) gequencht, mit Diethylether (3 × 50 ml) extrahiert und über wasserfreiem Na₂SO₄ getrocknet. Nach der Filtration wurde das Lösungsmittel unter Vakuum verdampft. Die Reinigung durch Flashchromatographie (CH₂Cl₂/*n*-Pentan, 1:3) lieferte Verbindung **23** (189 mg, 67%) als farbloses Öl.
**¹H-NMR (300 MHz, CDCl₃)** *δ*: 7,15 (m, 1 H), 8,89-8,93 (m, 1 H), 6,24 (s, 1 H), 5,31 (s, 1 H), 4,19 (q, *J =* 7,1 Hz, 2 H), 3,86 (s, 3 H), 3,63 (bs, 2 H), 1,27 (t, *J =* 7,1 Hz, 3 H). **¹³C**-**NMR (75 MHz, CDCl₃)** *δ*: 165,9, 154,3 (d, *J* = 243,6 Hz), 147,1 (d, *J* = 2,8 Hz), 136,2 (d, *J =* 0,8 Hz), 126,3 (d, *J* = 0,8 Hz), 120,0 (d, *J* = 21,9 Hz), 117,6, 117,3, 111,7 (d, *J* = 8,3 Hz), 61,1, 56,7, 26,9 (d, *J* = 2,9 Hz), 14,1.
**MS (70 eV, EI)** *m*/*z* (%): 283 (1) [M⁺], 237 (100), 209 (88), 192 (58), 166 (20), 149 (21), 133 (16), 121 (13), 99 (11).
**IR (ATR)** *ṽ* (cm⁻¹): 2969, 2359, 1738, 1503, 1385, 1342, 1294, 1226, 1215, 1084, 1013, 987, 954, 795, 764, 749, 667, 621, 615, 608, 603.
**HRMS (ESI) für C₁₃H₁₄FNO₅** (283,0856): 283,0845.

### Synthese von Methyl-3-(cyclohex-2-enyl)-5-nitrofuran-2-carboxylat (26):

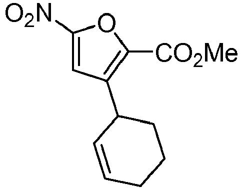

Methyl-5-nitrofuran-2-carboxylat **(24)** (171 mg, 1,0 mmol) in THF (2 ml) wurde zu einer Lösung von TMPZnCl·LiCl (**2**) (1,3 M in THF, 0,85 ml, 1,1 mmol) bei 25 °C zugegeben und das Reaktionsgemisch wurde dann bei dieser Temperatur während 30 Minuten gemäß **TP 2** gerührt. Nach Abkühlen auf -50 °C wurden 3-Bromcyclohexen (209 mg, 1,3 mmol) und CuCN·2LiCl (1,0 M Lösung in THF, 5 Tropfen) hinzugefügt und das so erhaltene Gemisch wurde während 1 Stunde bei dieser Temperatur gerührt. Das Reaktionsgemisch wurde mit einer gesättigten wässrigen NH₄Cl-Lösung (20 ml) gequencht, mit Diethylether (3 × 50 ml) extrahiert und über wasserfreiem Na₂SO₄ getrocknet. Nach der Filtration wurde das Lösungsmittel unter Vakuum verdampft. Die Reinigung durch Flashchromatographie (CH₂Cl₂/*n*-Pentan, 1:2) lieferte Verbindung **26** (179 mg, 72%) als gelbliches Öl.
**¹H-NMR (400 MHz, CDCl₃)** *δ*: 7,20 (s, 1 H), 5,94 (m, 1 H), 5,56 (m, 1 H), 4,10 (m, 1 H), 3,92 (s, 3 H), 2,07 (m, 3 H), 1,50-1,69 (m, 3 H).
**¹³C**-**NMR (100 MHz, CDCl₃)** *δ*: 157,5, 142,6, 133,9, 130,4, 126,2, 120,1, 52,8, 32,2, 29,0, 24,6, 20,5.
**MS (70 eV, EI)** *m*/*z* (%): 252 (2) [M⁺], 234 (100), 217 (55), 146 (10).
**IR (ATR)** *ṽ* (cm⁻¹): 2936,2356, 1729.35, 1629, 1594, 1532, 1502, 1435, 1398, 1338, 1288, 1226, 1206, 1110, 1091, 985, 925, 880, 848, 819, 799, 763, 725, 668, 634, 622. **HRMS (EI) für C₁₂H₁₃NO₅**) (251,0794): 251,0794.

### Synthese von 2-(3-(Trifluormethyl)phenyl)benzo[b]thiophen-3-carbaldehyd (29a):

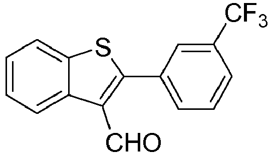

Benzo[b]thiophen-3-carbaldehyd **(27)** (162 mg, 1,0 mmol) in THF (2 ml) wurde zu einer Lösung von TMPZnCl·LiCl (**2**) (1,3 M in THF, 0,85 ml, 1,1 mmol) bei 25 °C zugegeben und das Reaktionsgemisch wurde dann bei dieser Temperatur während 30 Minuten gemäß **TP 2** gerührt. Pd(dba)₂ (17 mg, 3 mol%) und P(o-furyl)₃ (14 mg, 6 mol%), gelöst in THF (2 ml) und mit 3-lodbenzomethyltrifluorid (354 mg, 1,3 mmol, 1,3 Äquiv.) gemischt, wurden dann mittels einer Kanüle in das Reaktionsgemisch überführt. Das so erhaltene Gemisch wurde während 1 Stunde bei 25 °C gerührt. Das Reaktionsgemisch wurde dann mit einer gesättigten wässrigen NH₄Cl-Lösung (20 ml) gequencht, mit Diethylether (3 × 50 ml) extrahiert und über wasserfreiem Na₂SO₄ getrocknet. Nach der Filtration wurde das Lösungsmittel unter Vakuum verdampft. Die Reinigung durch Flashchromatographie (CH₂Cl₂/*n*-Pentan, 1:3) lieferte Verbindung **29a** (281 mg, 92%) als farblosen Feststoff.
**Schmp.:** 102,8 - 104,2 °C.
**¹H**-**NMR (400 MHz, CDCl₃)** δ: 10,02 (s, 1 H), 8,79 (m, 1 H), 7,45-7,87 (m, 7 H). **¹³C-NMR (100 MHz, CDCl₃) δ**: 185,9, 158,0, 138,0, 136,8, 133,7, 132,4, 131,5 (q, *J* (C-F) = 33,0 Hz), 130,7, 129,5, 127,0 (q, *J* (C-F) = 3,8 Hz), 126,6 (q, *J* (C-F) = 3,8 Hz), 126,5, 126,2, 123,5 (q, *J* (C-F) = 272,5 Hz), 121,7.
**MS (70 eV, EI)** *m*/*z* (%): 306 (97) [M⁺], 305 (100), 278 (12), 257 (13), 237 (28), 233 (18), 208 (29), 160 (13), 44 (40).
**IR (ATR)** *ṽ* (cm⁻¹): 3068, 2866, 2359, 1926, 1745, 1669, 1590, 1520, 1483, 1459, 1438, 1421, 1392, 1351, 1325, 1310, 1288, 1265, 1217, 1178, 1156, 1118, 1097, 1092, 1073, 1051, 1018, 1000, 994, 966, 947, 933, 907, 868, 863, 812, 773, 754, 733,703,679,653,641,633,620,608,603.
**HRMS (EI)** für **C₁₆H₉F₃OS** (306,0326): 306,0326.

### Synthese von 2-(4-Chlorphenyl)benzo[b]thiophen-3-carbaldehyd (29b):

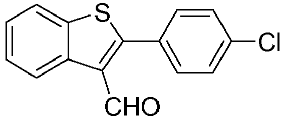

Benzo[b]thiophen-3-carbaldehyd **(27)** (162 mg, 1,0 mmol) in THF (2 ml) wurde zu einer Lösung von TMPZnCl·LiCl (**2**) (1,3 M in THF, 0,85 ml, 1,1 mmol) bei 25 °C zugegeben und das Reaktionsgemisch wurde dann bei dieser Temperatur während 30 Minuten gemäß **TP 2** gerührt. Pd(dba)₂ (17 mg, 3 mol%) und P(o-furyl)₃ (14 mg, 6 mol%), gelöst in THF (2 ml) und mit 1-Chlor-4-iodbenzol (310 mg, 1,3 mmol, 1,3 Äquiv.) gemischt, wurden dann mittels einer Kanüle in das Reaktionsgemisch überführt. Das so erhaltene Gemisch wurde während 2 Stunden bei 25 °C gerührt. Das Reaktionsgemisch wurde dann mit einer gesättigten wässrigen NH₄Cl-Lösung (20 ml) gestoppt, mit Diethylether (3 × 50 ml) extrahiert und über wasserfreiem Na₂SO₄ getrocknet. Nach der Filtration wurde das Lösungsmittel unter Vakuum verdampft. Die Reinigung durch Flashchromatographie (CH₂Cl₂/*n*-Pentan, 1:3) lieferte Verbindung **29b** (236 mg, 87%) als farblosen Feststoff.
**Schmp.:** 99,7-101,4 °C.
**¹H**-**NMR (300 MHz, CDCl₃)** δ: 10,02 (s, 1 H), 8,76 (d, *J* = 8,0 Hz, 1 H), 7,83 (d, *J* = 8,0 Hz, 1 H), 7,42-7,54 (m, 6 H).
**¹³C-NMR (75 MHz, CDCl₃) δ:** 186,2, 158,9, 137,8, 136,9, 136,4, 131,6, 130,3, 130,0, 129,2, 126,4, 126,0, 125,2, 121,6.
**MS (70 eV, EI)** *m*/*z* (%): 272 (100) [M⁺], 237 (54), 208 (34), 165 (12), 118 (20), 104 (23).
**IR (ATR)** *ṽ* (cm⁻¹): 3054, 2969, 2867, 2362, 1947, 1739, 1671, 1590, 1562, 1517, 1482, 1457, 1431, 1407, 1397, 1346, 1265, 1218, 1187, 1161, 1135, 1109, 1091, 1050, 1020, 1012, 971, 952, 938, 846, 830, 813, 748, 723, 716, 710, 698, 667, 638, 616,610,603.
**HRMS (EI) für C₁₅H₉ClOS** (272,0063): 272,0057.

### Synthese von 2-(Phenylethinyl)benzo[b]thiophen-3-carbaldehyd (29c):

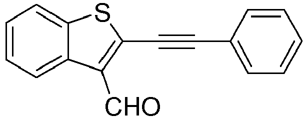

Benzo[b]thiophen-3-carbaldehyd (27) (162 mg, 1,0 mmol) in THF (2 ml) wurde zu einer Lösung von TMPZnCl·LiCl (**2**) (1,3 M in THF, 0,85 ml, 1,1 mmol) bei 25 °C zugegeben und das Reaktionsgemisch wurde dann bei dieser Temperatur während 30 Minuten gemäß **TP 2** gerührt. In trockenem THF (2 ml) gelöstes I₂ (381 mg, 1,5 mmol) wurde dann tropfenweise hinzugefügt und das so erhaltene Gemisch wurde während 0,5 Stunden gerührt. Zu der Lösung des frisch in situ hergestellten 2-lodbenzo[b]thiophen-3-carbaldehyds wurden NEt₃ (7 ml), Cul (8 mg, 4 mol%), Pd(dba)₂ (17 mg, 3 mol%) und P(o-furyl)₃ (14 mg, 6 mol%) in THF (2 ml) und Phenylacetylen (254 mg, 1,5 mol, 1,5 Äquiv.) nacheinander langsam hinzugefügt. Das Reaktionsgemisch wurde bei Raumtemperatur während 2 Stunden gerührt. Das Reaktionsgemisch wurde mit einer gesättigten wässrigen Na₂S₂O₃-Lösung (10 ml) und mit einer gesättigten wässrigen NH₄Cl-Lösung (20 ml) gestoppt, mit Diethylether (3 × 50 ml) extrahiert und über wasserfreiem Na₂SO₄ getrocknet. Nach der Filtration wurde das Lösungsmittel unter Vakuum verdampft. Die Reinigung durch Flashchromatographie (CH₂Cl₂/*n*-Pentan, 1:2) lieferte Verbindung **29c** (165 mg, 63%) als gelblichen Feststoff.
**Schmp.:** 104,9 - 106,5 °C.
**¹H-NMR (400 MHz, CDCl₃)** *δ*: 10,47 (s, 1 H), 8,69 (m, 1 H), 7,77 (m, 1 H), 7,60 (m, 2 H), 7,38-7,51 (m, 5 H).
**¹³C-NMR (100 MHz, CDCi₃)** *δ*: 185,6, 138,9, 138,5, 135,9, 135,2, 131,8, 129,8, 128,6, 126,8, 126,5, 124,9, 121,6, 121,3, 102,9, 80,0.
**MS (70 eV, EI)** *m*/*z* (%): 262 (100) [M⁺], 234 (38), 232 (13), 202 (11), 189 (13).
**IR (ATR)** *ṽ* (cm⁻¹): 2969, 2832, 2359, 2340, 2203, 1739, 1661, 1587, 1569, 1507, 1481, 1458, 1442, 1427, 1361, 1316, 1293, 1250, 1229, 1216, 1177, 1162, 1141, 1119, 1070, 1059, 1043, 1015, 997, 953, 918, 868, 748, 737, 697, 687, 668, 630, 621, 616,610.
**HRMS (EI) für C₁₇H₁₀OS** (262,0452): 262,0459.

## Patentansprüche

1. Reagenz der allgemeinen Formel
R¹R²N-ZnY LiY (I)
worin ist
R¹, R² unabhängig ausgewählt aus H, substituiertem oder unsubstituiertem Aryl oder Heteroaryl mit ein oder mehr Heteroatomen, geradem, verzweigtem oder zyklischem substituiertem oder unsubstituiertem Alkyl, Alkenyl, Alkinyl oder deren Silylderivaten; wobei R¹ und R² zusammen Teil einer zyklischen oder polymeren Struktur sein können, worin mindestens einer der Reste R¹ und R² nicht H ist;
Y ausgewählt aus der Gruppe mit F; Cl; Br; I; CN; SCN; NCO; HalOₙ, wobei n gleich 3 oder 4 ist und Hal ausgewählt ist aus Cl, Br und I; NO₃; BF₄; PF₆; H; einem Carboxylat der allgemeinen Formel R^{x}CO₂; einem Alkoholat der allgemeinen Formel OR^{x}; einem Thiolat der allgemeinen Formel SR^{x}; R^{x}P(O)O₂; oder SCOR^{x}; oder SCSR^{x}; OₙSR^{x}, wobei n gleich 2 oder 3; oder NOₙ, wobei n gleich 2 oder 3; und deren Derivaten; wobei R^{x} ein substituiertes oder unsubstituiertes Aryl ist oder Heteroaryl mit ein oder mehr Heteroatomen, gerades, verzweigtes oder zyklisches substituiertes oder unsubstituiertes Alkyl, Alkenyl, Alkinyl oder deren Derivate oder H; oder als Addukt mit einem Lösungsmittel.

2. Reagenz nach Anspruch 1, wobei R¹, R² zyklisch sind und mit R³ und R⁴ substituiert, die unabhängig ausgewählt sind aus H, substituiertem oder unsubstituiertem Aryl oder Heteroaryl mit ein oder mehr Heteroatomen, geradem, verzweigtem oder zyklischem substituiertem oder unsubstituiertem Alkyl, Alkenyl, Alkinyl oder deren Silylderivaten; und R¹ und R² zusammen oder R³ und R⁴ zusammen einen Teil einer zyklischen oder polymeren Struktur bilden können; wobei mindestens einer der Reste R¹ und R² und mindestens einer der Reste R³ und R⁴ nicht H ist.

3. Reagenz nach Anspruch 1 oder 2, worin das R¹R²N-ZnY LiY der Formel (I) gleich 2,2,6,6-Tetramethylpiperid-Zinkchlorid-Lithiumchlorid ist.

4. Lösung des Reagenzes nach Anspruch 1 bis 3 in einem Lösungsmittel.

5. Lösung des Reagenzes nach Anspruch 1 bis 4, wobei das Lösungsmittel polar und aprotisch ist.

6. Lösung nach Anspruch 5, wobei das Lösungsmittel ausgewählt ist aus zyklischen, geraden oder verzweigten Mono- oder Polyethern, Thioethern, Aminen, Phosphinen und deren Derivaten, die ein oder mehr weitere Heteroatome enthalten, ausgewählt aus O, N, S und P, vorzugsweise Tetrahydrofuran (THF), 2-Methyl-tetrahydrofuran, Dibutylether, Diethylether, tert.-Butylmethylether, Dimethoxyethan, Dioxanen, vorzugsweise 1,4-Dioxan, Triethylamin, Ethyldiisopropylamin, Dimethylsulfid, Dibutylsulfid; zyklischen Amiden, vorzugsweise N-Methyl-2-pyrrolidon (NMP), N-Ethyl-2-pyrrolidon (NEP), N-Butyl-2-pyrrolidon (NBP); zyklischen, geraden oder verzweigten Alkanen und/oder Alkenen, wobei ein oder mehr Wasserstoffatome durch ein Halogenatom ersetzt sind, vorzugsweise Dichlormethan, 1,2-Dichlorethan, CCl₄; Harnstoffderivaten, vorzugsweise N,N'-Dimethylpropylenharnstoff (DMPU); aromatischen, heteroaromatischen oder aliphatischen Kohlenwasserstoffen, vorzugsweise Benzol, Toluol, Xylol, Pyridin, Pentan, Cyclohexan, Hexan, Heptan; Hexamethylphosphortriamid (HMPA), CS₂, oder deren Kombinationen.

7. Verfahren zur Herstellung eines Reagenzes der allgemeinen Formel
R¹R²N-ZnY LiY (I)
worin ist
R¹, R² unabhängig ausgewählt aus H, substituiertem oder unsubstituiertem Aryl oder Heteroaryl mit ein oder mehr Heteroatomen, geradem, verzweigtem oder zyklischem substituiertem oder unsubstituiertem Alkyl, Alkenyl, Alkinyl oder deren Siliciumderivaten; und R¹ und R² zusammen Teil einer zyklischen oder polymeren Struktur sein können; worin mindestens einer der Reste R¹ und R² nicht H ist;
Y ausgewählt aus der Gruppe mit F; Cl; Br; I; CN; SCN; NCO; HalOₙ, wobei n gleich 3 oder 4 und Hal ausgewählt ist aus Cl, Br und I; NO₃; BF₄; PF₆; H; einem Carboxylat der allgemeinen Formel R^{x}CO₂; einem Alkoholat der allgemeinen Formel OR^{x}; einem Thiolat der allgemeinen Formel SR^{x}; R^{x}P(O)O₂; oder SCOR^{x}; oder SCSR^{x}; OₙSR^{x}, wobei n gleich 2 oder 3; oder NOₙ, wobei n gleich 2 oder 3; und einem Derivat davon, wobei R^{x} ein substituiertes oder unsubstituiertes Aryl oder Heteroaryl mit ein oder mehr Heteroatomen, gerades, verzweigtes oder zyklisches substituiertes oder unsubstituiertes Alkyl, Alkenyl, Alkinyl oder deren Derivate oder H ist;
umfassend das Umsetzen in einem Lösungsmittel von R¹R²N-H mit R^{x}Li in Gegenwart von ZnY₂, und X wie oben Y definiert ist.

8. Verfahren nach Anspruch 7, wobei X und Y unabhängig oder beide Cl, Br oder I und vorzugsweise Cl sind.

9. Verfahren nach einem der Ansprüche 7 oder 8, wobei das Lithiumorganyl-Reagenz *sek*-Butyl-Li ist.

10. Verfahren nach einem der Ansprüche 7 bis 9, wobei das Lösungsmittel ausgewählt ist aus zyklischen, geraden oder verzweigten Mono- oder Polyethern, Thioethern, Aminen, Phosphinen und deren Derivaten, die ein oder mehr weitere Heteroatome enthalten, ausgewählt aus O, N, S und P, vorzugsweise Tetrahydrofuran (THF), 2-Methyltetrahydrofuran, Dibutylether, Diethylether, tert.-Butylmethylether, Dimethoxyethan, Dioxanen, vorzugsweise 1,4-Dioxan, Triethylamin, Ethyldiisopropylamin, Dimethylsulfid, Dibutylsulfid; zyklischen Amiden, vorzugsweise N-Methyl-2-pyrrolidon (NMP), N-Ethyl-2-pyrrolidon (NEP), N-Butyl-2-pyrrolidon (NBP); zyklischen, geraden oder verzweigten Alkanen und/oder Alkenen, wobei ein oder mehr Wasserstoffatome durch ein Halogenatom ersetzt sind, vorzugsweise Dichlormethan, 1,2-Dichlorethan, CCl₄; Harnstoffderivaten, vorzugsweise N,N'-Dimethylpropylenharnstoff (DMPU); aromatischen, heteroaromatischen oder aliphatischen Kohlenwasserstoffen, vorzugsweise Benzol, Toluol, Xylol, Pyridin, Pentan, Cyclohexan, Hexan, Heptan; Hexamethylphosphortriamid (HMPA), CS₂, oder deren Kombinationen.

11. Verwendung des Reagenzes nach Anspruch 1 bis 6 in einer Umsetzung mit einem Elektrophil.

12. Verwendung des Reagenzes nach Anspruch 1 bis 6 zur Deprotonierung eines beliebigen Substrats, das stabilisierte oder unstabilisierte Carbanionen bilden kann.

13. Verwendung des Reagenzes nach Anspruch 1 bis 6 zur Herstellung von
• 3,6-Dichlor-4-iodpyridazin,
• (3,6-Dichlorpyradizin-4-yl)(4-fluorphenyl)methanon,
• 3,6-Dichlor-4-(3-(trifluormethyl)phenyl)pyridazin,
• 4,6-Dichlor-5-iod-pyrimidin,
• (4,6-Dichlorpyrimidin-5-yl)(furan-2-yl)methanon,
• 5-Allyl-4,6-dichlorpyrimidin,
• 3,5-Dichlor-2-iodpyrazin,
• Ethyl-4-(3,5-dichlorpyrazin-2-yl)benzoat,
• Ethyl-2-((3,5-dichlorpyrazin-2-yl)methyl)acrylat,
• 8-(4-Chlorphenyl)-1,3,7-trimethyl-1 H-purin-2,6(3H,7H)-dion,
• Ethyl-2-((1,3,7-trimethyl-2,6-dioxo-2,3,6,7-tetrahydro-1H-purin-8-yl)methyl)acrylat,
• Ethyl-2',6'-difluor-3'-nitrobiphenyl-4-carboxylat,
• (2,6-Difluor-3-nitrophenyl)(phenyl)methanon,
• 1,3-Difluor-2-iod-4-nitrobenzol,
• 2-Chlor-4-cyclohex-2-enyl-3-nitropyridin,
• Ethyl-2-(6-fluor-3-methoxy-2-nitrobenzyl)acrylat,
• Methyl-3-(cyclohex-2-enyl)-5-nitrofuran-2-carboxylat,
• 2-(3-(Trifluormethyl)phenyl)benzo[b]thiophen-3-carbaldehyd,
• 2-(4-Chlorphenyl)benzo[b]thiophen-3-carbaldehyd oder
• 2-(Phenylethinyl)benzo[b]thiophen-3-carbaldehyd.

## Claims

1. A reagent of the general formula
R¹R²N-ZnY LiY (I)
wherein
R¹, R² are, independently, selected from H, substituted or unsubstituted aryl or heteroaryl with one or more heteroatoms, linear, branched or cyclic substituted or unsubstituted alkyl, alkenyl, alkynyl, or silyl derivatives thereof; wherein R¹ and R² together can be part of a cyclic or polymeric structure; wherein at least one of the residues R¹ and R² is not H;
Y is selected from the group with F; Cl; Br; I; CN; SCN; NCO; HalOₙ, wherein n = 3 or 4, and Hal is selected from Cl, Br and I; NO₃; BF₄; PF₆; H; a carboxylate of the general formula R^{x}CO₂; an alcoholate of the general formula OR^{x}; a thiolate of the general formula SR^{x}; R^{x}P(O)O₂; or SCOR^{x}; or SCSR^{x}; OₙSR^{x}, wherein n = 2 or 3; or NOₙ, wherein n = 2 or 3; and derivatives thereof; wherein R^{x} is a substituted or unsubstituted aryl or heteroaryl with one or more heteroatoms, linear, branched or cyclic substituted or unsubstituted alkyl, alkenyl, alkynyl, or derivatives thereof, or H; or as adduct with a solvent.

2. A reagent according to claim 1, wherein R¹, R² are cyclic and substituted by R³ and R⁴ that are, independently, selected from H, substituted or unsubstituted aryl or heteroaryl with one or more heteroatoms, linear, branched or cyclic substituted or unsubstituted alkyl, alkenyl, alkynyl, or silyl derivatives thereof; and R¹ and R² together, or R³ and R⁴ together, can form part of a cyclic or polymeric structure; wherein at least one of the residues R¹ and R² and at least one of the residues R³ and R⁴ is not H.

3. A reagent according to claim 1 or 2, wherein R¹R²N-ZnY LiY of the formula (I) is equal to 2,2,6,6-tetramethylpiperide zinc chloride lithium chloride.

4. A solution of the reagent according to claim 1. to 3 in a solvent.

5. A solution of the reagent according to claim 1 to 4, wherein the solvent is polar and aprotic.

6. A solution according to claim 5, wherein the solvent is selected from cyclic, linear or branched mono or polyethers, thioethers, amines, phosphines, and derivatives thereof containing one or more additional heteroatoms selected from O, N, S and P, preferably tetrahydrofuran (THF), 2-methyltetrahydrofuran, dibutyl ether, diethyl ether, tert-butylmethyl ether, dimethoxyethane, dioxanes, preferably 1,4-dioxane, triethylamine, ethyldiisopropyl amine, dimethylsulphide, dibutylsulphide; cyclic amides, preferably N-methyl-2-pyrrolidone (NMP), N-ethyl-2-pyrrolidone (NEP), N-butyl-2-pyrrolidone (NBP); cyclic, linear or branched alkanes and/or alkenes, wherein one or more hydrogen atoms are replaced by a halogen atom, preferably dichloromethane, 1,2-dichloroethane, CCl₄; urea derivatives, preferably N,N'-dimethylpropylene urea (DMPU); aromatic, heteroaromatic or aliphatic hydrocarbons, preferably benzene, toluene, xylene, pyridine, pentane, cyclohexane, hexane, heptane; hexamethylphosphorus triamide (HMPA), CS₂; or combinations thereof.

7. A process for the preparation of a reagent having the general formula
R¹R²N-ZnY LiY (I)
wherein
R¹, R² are, independently, selected from H, substituted or unsubstituted aryl or heteroaryl with one or more heteroatoms, linear, branched or cyclic substituted or unsubstituted alkyl, alkenyl, alkynyl, or silicon derivatives thereof; and R¹ and R² together can be part of a cyclic or polymeric structure; wherein at least one of the residues R¹ and R² is not H;
Y is selected from the group with F; Cl; Br; I; CN; SCN; NCO; HalOₙ, wherein n = 3 or 4, and Hal is selected from Cl, Br and I; NO₃; BF₄; PF₆; H; a carboxylate of the general formula R^{x}CO₂ ; an alcoholate of the general formula OR^{x}; a thiolate of the general formula SR^{x} ; R^{x}P(O)O₂; or SCOR^{x} ; or SCSR^{x}; OₙSR^{x}, wherein n = 2 or 3; or NOₙ, wherein n = 2 or 3; and a derivative thereof; wherein R^{x} is a substituted or unsubstituted aryl or heteroaryl with one or more heteroatoms, linear, branched or cyclic substituted or unsubstituted alkyl, alkenyl, alkynyl, or derivatives thereof, or H;
comprising the reaction in a solvent of R¹R²N-H with R^{x}Li in the presence of ZnY₂, and X is defined as Y above.

8. A process according to claim 7, wherein X and Y are independently or both Cl, Br or I, and preferably Cl.

9. A process according to one of claims 7 or 8, wherein the lithium organyl reagent is sec-butyl-Li.

10. A process according to one of claims 7 to 9, wherein the solvent is selected from cyclic, linear or branched mono or polyethers, thioethers, amines, phosphines, and derivatives thereof containing one or more additional heteroatoms selected from O, N, S and P, preferably tetrahydrofuran (THF), 2-methyltetrahydrofuran, dibutyl ether, diethyl ether, tert-butylmethyl ether, dimethoxyethane, dioxanes, preferably 1,4-dioxane, triethylamine, ethyldiisopropylamine, dimethylsulphide, dibutylsulphide; cyclic amides, preferably N-methyl-2-pyrrolidone (NMP), N-ethyl-2-pyrrolidone (NEP), N-butyl-2-pyrrolidone (NBP) ; cyclic, linear or branched alkanes and/or alkenes, wherein one or more hydrogen atoms are replaced by a halogen atom, preferably dichloromethane, 1,2-dichloroethane, CCl₄ ; urea derivatives, preferably N,N'-dimethylpropylene urea (DMPU); aromatic, heteroaromatic or aliphatic hydrocarbons, preferably benzene, toluene, xylene, pyridine, pentane, cyclohexane, hexane, heptane; hexamethylphosphorus triamide (HMPA), CS₂; or combinations thereof.

11. Use of the reagent according to claim 1 to 6 in a reaction with an electrophile.

12. Use of the reagent according to claim 1 to 6 for the deprotonation of any substrate which can form stabilized or unstabilized carbanions.

13. Use of the reagent according to claim 1 to 6 for the preparation of
• 3,6-dichloro-4-iodopyridazine,
• (3,6-dichloropyradizin-4-yl) (4-fluorophenyl)methanone,
• 3,6-dichloro-4-(3-(trifluoromethyl)phenyl)pyridazine,
• 4,6-dichloro-5-iodo-pyrimidine,
• (4,6-dichloropyrimidin-5-yl)(furan-2-yl)methanone,
• 5-allyl-4,6-dichloropyrimidine,
• 3,5-dichloro-2-iodopyrazine,
• ethyl-4-(3,5-dichloropyrazin-2-yl)benzoate,
• ethyl-2-((3,5-dichloropyrazin-2-yl)methyl)acrylate,
• 8-(4-chlorophenyl)-1,3,7-trimethyl-1H-purine-2,6(3H,7H)-dione,
• ethyl-2-((1,3,7-trimethyl-2,6-dioxo-2,3,6,7-tetrahydro-1H-purin-8-yl)methyl)acrylate,
• ethyl-2',6'-difluoro-3'-nitrobiphenyl-4-carboxylate,
• (2,6-difluoro-3-nitrophenyl)(phenyl)methanone,
• 1,3-difluoro-2-iodo-4-nitrobenzene,
• 2-chloro-4-cyclohex-2-enyl-3-nitropyridine,
• ethyl-2-(6-fluoro-3-methoxy-2-nitrobenzyl)acrylate,
• methyl-3-(cyclohex-2-enyl)-5-nitrofuran-2-carboxylate,
• 2-(3-(trifluoromethyl)phenyl)benzo[b]thiophene-3-carbaldehyde,
• 2-(4-chlorophenyl)benzo[b]thiophene-3-carbaldehyde or
• 2-(phenylethynyl)benzo[b]thiophene-3-carbaldehyde.

## Revendications

1. Réactif de formule générale
R¹R²N-ZnY LiY (I)
dans laquelle
- R¹ et R² représentent des entités choisies, indépendamment, parmi
- un atome d'hydrogène,
- un groupe aryle ou hétéroaryle comportant un ou plusieurs hétéroatome(s), avec ou sans substituant(s),
- et un groupe alkyle, alcényle ou alcynyle, à chaîne droite, ramifiée ou cyclique, avec ou sans substituant(s), ou un dérivé silyle d'un tel groupe,
étant entendu que R¹ et R² peuvent aussi représenter conjointement une partie d'une structure cyclique ou polymère,
et étant entendu que l'une au moins des entités représentées par R¹ et R² n'est pas un atome d'hydrogène ;
- et Y représente une entité choisie dans l'ensemble formé par
- celles symbolisées par F, Cl, Br, I, CN, SCN, NCO, HalOₙ où l'indice n vaut 3 ou 4 et Hal représente un atome de chlore, de brome ou d'iode, NO₃, BF₄, PF₆ ou H,
- et un carboxylate de formule générale R^{x}CO₂, un alcoolate de formule générale OR^{x}, un thiolate de formule générale SR^{x}, ou une entité symbolisée par R^{x}P(O)O₂, SCOR^{x}, SCSR^{x}, OₙSR^{x} où l'indice n vaut 2 ou 3, ou NOₙ où l'indice n vaut 2 ou 3, ainsi que les dérivés de telles entités,
étant entendu que R^{x} représente un groupe aryle ou hétéroaryle comportant un ou plusieurs hétéroatome(s), avec ou sans substituant(s), un groupe alkyle, alcényle ou alcynyle, à chaîne droite, ramifiée ou cyclique, avec ou sans substituant(s), ou un dérivé d'un tel groupe, ou encore un atome d'hydrogène ;
lequel réactif peut être à l'état d'adduit formé avec un solvant.

2. Réactif conforme à la revendication 1, dans lequel les entités que représentent R¹ et R² sont des groupes cycliques porteurs de substituants symbolisés par R³ et R⁴, lesquels symboles représentent des entités choisies, indépendamment, parmi un atome d'hydrogène, un groupe aryle ou hétéroaryle comportant un ou plusieurs hétéroatome(s), avec ou sans substituant(s), et un groupe alkyle, alcényle ou alcynyle, à chaîne droite, ramifiée ou cyclique, avec ou sans substituant(s), ou un dérivé silyle d'un tel groupe, étant entendu que R¹ et R², ou R³ et R⁴, peuvent aussi représenter conjointement une partie d'une structure cyclique ou polymère, et étant entendu que l'une au moins des entités représentées par R¹ et R², ainsi que l'une au moins des entités représentées par R³ et R⁴, n'est pas un atome d'hydrogène.

3. Réactif conforme à la revendication 1 ou 2, dans lequel le composé de formule (I) R¹R²N-ZnY LiY est du chlorure de 2,2,6,6-tétraméthyl-pipéridyl-zinc-chlorure de lithium.

4. Solution d'un réactif conforme à l'une des revendications 1 à 3 dans un solvant.

5. Solution de réactif, conforme à l'une des revendications 1 à 4, dans laquelle le solvant est polaire et aprotique.

6. Solution conforme à la revendication 5, dans laquelle le solvant est choisi parmi les suivants :
- mono- ou poly-éthers, -thioéthers, -amines et -phosphines, à chaîne droite, ramifiée ou cyclique, et leurs dérivés, qui comportent un ou plusieurs autre(s) hétéroatome(s) choisi(s) parmi les atomes d'oxygène, d'azote, de soufre et de phosphore, et de préférence les tétrahydrofurane (THF), 2-méthyl-tétrahydrofurane, dibutyl-éther, diéthyl-éther, tertiobutyl-méthyl-éther, diméthoxy-éthane, dioxanes, de préférence le 1,4-dioxane, triéthyl-amine, éthyl-diisopropyl-amine, diméthyl-sulfure et dibutyl-sulfure ;
- amides cycliques, de préférence les N-méthyl-2-pyrrolidone (NMP), N-éthyl-2-pyrrolidone (NEP) et N-butyl-2-pyrrolidone (NBP) ;
- alcanes et/ou alcènes à chaîne droite, ramifiée ou cyclique, dont un ou plusieurs atome(s) d'hydrogène est ou sont remplacé(s) par un ou des atome(s) d'halogène, de préférence les dichloro-méthane, 1,2-dichloro-éthane et tétrachloro-méthane ;
- dérivés d'urée, de préférence la N,N'-diméthyl-propylène-urée (NMPU) ;
- hydrocarbures aromatiques, hétéroaromatiques ou aliphatiques, de préférence les benzène, toluène, xylène, pyridine, pentane, cyclohexane, hexane et heptane ;
- hexaméthyl-phosphotriamide (HMPA), disulfure de carbone (CS₂) ; et les combinaisons de ces solvants.

7. Procédé de préparation d'un réactif de formule générale :
R¹R²N-ZnY LiY (I)
dans laquelle
- R¹ et R² représentent des entités choisies, indépendamment, parmi
- un atome d'hydrogène,
- un groupe aryle ou hétéroaryle comportant un ou plusieurs hétéroatome(s), avec ou sans substituant(s),
- et un groupe alkyle, alcényle ou alcynyle, à chaîne droite, ramifiée ou cyclique, avec ou sans substituant(s), ou un dérivé silyle d'un tel groupe,
étant entendu que R¹ et R² peuvent aussi représenter conjointement une partie d'une structure cyclique ou polymère,
et étant entendu que l'une au moins des entités représentées par R¹ et R² n'est pas un atome d'hydrogène ;
- et Y représente une entité choisie dans l'ensemble formé par
- celles symbolisées par F, Cl, Br, I, CN, SCN, NCO, HalOₙ où l'indice n vaut 3 ou 4 et Hal représente un atome de chlore, de brome ou d'iode, NO₃, BF₄, PF₆ ou H,
- et un carboxylate de formule générale R^{x}CO₂, un alcoolate de formule générale OR^{x}, un thiolate de formule générale SR^{x}, ou une entité symbolisée par R^{x}P(O)O₂, SCOR^{x}, SCSR^{x}, OₙSR^{x} où l'indice n vaut 2 ou 3, ou NOₙ où l'indice n vaut 2 ou 3, ainsi que les dérivés de telles entités,
étant entendu que R^{x} représente un groupe aryle ou hétéroaryle comportant un ou plusieurs hétéroatome(s), avec ou sans substituant(s), un groupe alkyle, alcényle ou alcynyle, à chaîne droite, ramifiée ou cyclique, avec ou sans substituant(s), ou un dérivé d'un tel groupe, ou encore un atome d'hydrogène ;
lequel procédé comporte le fait de faire réagir, dans un solvant, un composé de formule R¹R²NH avec un composé de formule R^{x}Li, en présence d'un composé de formule ZnY₂, étant entendu que le symbole X a la même signification que celle donnée ci-dessus pour Y.

8. Procédé conforme à la revendication 7, dans lequel X et Y représentent, indépendamment ou tous les deux, des atomes de chlore, de brome ou d'iode, et de préférence, des atomes de chlore.

9. Procédé conforme à la revendication 7 ou 8, dans lequel le réactif organo-lithien est du *sec*-butyl-lithium.

10. Procédé conforme à l'une des revendications 7 à 9, dans lequel le solvant est choisi parmi les suivants :
- mono- ou poly-éthers, -thioéthers, -amines et -phosphines, à chaîne droite, ramifiée ou cyclique, et leurs dérivés, qui comportent un ou plusieurs autre(s) hétéroatome(s) choisi(s) parmi les atomes d'oxygène, d'azote, de soufre et de phosphore, et de préférence les tétrahydrofurane (THF), 2-méthyl-tétrahydrofurane, dibutyl-éther, diéthyl-éther, tertiobutyl-méthyl-éther, diméthoxy-éthane, dioxanes, de préférence le 1,4-dioxane, triéthyl-amine, éthyl-diisopropyl-amine, diméthyl-sulfure et dibutyl-sulfure ;
- amides cycliques, de préférence les N-méthyl-2-pyrrolidone (NMP), N-éthyl-2-pyrrolidone (NEP) et N-butyl-2-pyrrolidone (NBP) ;
- alcanes et/ou alcènes à chaîne droite, ramifiée ou cyclique, dont un ou plusieurs atome(s) d'hydrogène est ou sont remplacé(s) par un ou des atome(s) d'halogène, de préférence les dichloro-méthane, 1,2-dichloro-éthane et tétrachloro-méthane ;
- dérivés d'urée, de préférence la N,N'-diméthyl-propylène-urée (NMPU) ;
- hydrocarbures aromatiques, hétéroaromatiques ou aliphatiques, de préférence les benzène, toluène, xylène, pyridine, pentane, cyclohexane, hexane et heptane ;
- hexaméthyl-phosphotriamide (HMPA), disulfure de carbone (CS₂) ; et les combinaisons de ces solvants.

11. Utilisation d'un réactif conforme à l'une des revendications 1 à 6 dans une réaction avec un composé électrophile.

12. Utilisation d'un réactif conforme à l'une des revendications 1 à 6 pour la déprotonation d'un substrat qui peut former des carbanions stabilisés ou non-stabilisés.

13. Utilisation d'un réactif conforme à l'une des revendications 1 à 6 pour la préparation de l'un des composés suivants :
- 3,6-dichloro-4-iodo-pyridazine
- (3,6-dichloro-pyridazin-4-yl)(4-fluoro-phényl)méthanone
- 3,6-dichloro-4-[3-(trifluoro-méthyl)-phényl]-pyridazine
- 4,6-dichloro-5-iodo-pyrimidine
- (4,6-dichloro-pyrimidin-5-yl)(furan-2-yl)méthanone
- 5-allyl-4,6-dichloro-pyrimidine
- 3,5-dichloro-2-iodo-pyridazine
- 4-(3,5-dichloro-pyrazin-2-yl)-benzoate d'éthyle
- 2-[(3,5-dichloro-pyrazin-2-yl)-méthyl]-acrylate d'éthyle
- 8-(4-chloro-phényl)-1,3,7-triméthyl-1H-purine-2,6(3H,7H)-dione
- 2-[(1,3,7-triméthyl-2,6-dioxo-2,3,6,7-tétrahydro-1H-purin-8-yl)-méthyl]-acrylate d'éthyle
- 2',6'-difluoro-3'-nitro-biphényle-4-carboxylate d'éthyle
- (2,6-difluoro-3-nitro-phényl)(phényl)méthanone
- 1,3-difluoro-2-iodo-4-nitro-benzène
- 2-chloro-4-(cyclohex-2-ènyl)-3-nitro-pyridine
- 2-(6-fluoro-3-méthoxy-2-nitro-benzyl)-acrylate d'éthyle
- 3-(cyclohex-2-ènyl)-5-nitro-furane-2-carboxylate de méthyle
- 2-[3-(trifluoro-méthyl)-phényl]-benzo[b]thiophène-3-carbaldéhyde
- 2-(4-chloro-phényl)-benzo[b]thiophène-3-carbaldéhyde
- 2-(phényl-éthynyl)-benzo[b]thiophène-3-carbaldéhyde.
